# EUROPEAN PATENT SPECIFICATION

(11) **EP 4 297 630 B1**
(45) Date of publication and mention of the grant of the patent: **17.06.2026**
(21) Application number: 22760261.2
(22) Date of filing: 22.02.2022
(51) Int. Cl.: A61F 9/00, A61B 3/16, G02C 11/00, A61F 9/02, G02C 7/04

(54) **DEVICE FOR REMOTE OPTICAL MONITORING OF INTRAOCULAR PRESSURE**
VORRICHTUNG ZUR OPTISCHEN FERNÜBERWACHUNG DES AUGENINNENDRUCKS
DISPOSITIF DE SURVEILLANCE OPTIQUE À DISTANCE DE LA PRESSION INTRAOCULAIRE

(30) Priority: 24.02.2021 US 202163152844 P
(43) Date of publication of application: 03.01.2024
(73) Proprietor: Smartlens, Inc., Mountain View, California 94043 (US)
(72) Inventor: DANA, Aykutlu, Mountain View, California 94043 (US); YAZICI, Ahmet Taylan, Mountain View, California 94043 (US); AGAOGLU, Sevda, Mountain View, California 94043 (US); KOMBAN, Savas, Mountain View, California 94043 (US); BADAY, Murat, Mountain View, California 94043 (US)
(74) Representative: Mathys & Squire
(86) International application number: PCT/US2022/017224
(87) International publication number: WO 2022/182629

(56) References cited:
- WO-A1-2017/156050
- WO-A1-2018/227190
- WO-A1-2019/211217
- US-A1- 2016 270 656
- US-A1- 2017 165 439
- US-A1- 2018 296 390
- US-A1- 2020 138 669
- US-A1- 2020 229 973
- US-B2- 7 981 097
- US-B2- 7 981 097

## Description

### BACKGROUND

1. Field of the Invention. The present disclosure is related to a system and methods of using a wearable optical imaging sensor system for measuring intraocular pressure and dispensing medication to treat the same.

Glaucoma is the second most common cause of blindness in the global world. It is a multifactorial disease with several risk factors, of which intraocular pressure (IOP) is the most important. IOP measurements are used for glaucoma diagnosis and patient monitoring. IOP has wide diurnal fluctuation, and is dependent on body posture, so the occasional measurements done by the eye care expert in a clinic can be misleading.

Previously (US20160015265A1, 2018), an implantable microfluidic device has been proposed for intraocular pressure monitoring, that can be used for glaucoma diagnosis. Later, a wearable device was demonstrated (Lab on a Chip, 2018, 18, 3471-3483) to serve the same purpose, however without needing implantation. In these previous studies, it was established that intraocular pressure increases results in bulging of the cornea and consequently changes in the radius of curvature.

In literature, it is shown that the IOP changes affect the corneal topography, causing changes in corneal radius and apex height with respect to the corneal periphery. If the corneal topography can be measured accurately, the 4 micrometer change in corneal radius per 1 mmHg IOP change can be monitored and IOP value can be inferred.

Thus there remains a need for an IOP measuring device that can take multiple measurements of a patient eye throughout the day as the patient goes through their normal routine.

There is also a need for a device that has sufficient sensitivity to take measurements to produce reliable data for accurate diagnosis.

There is still further a need for such a device to operate in a manner that does not interfere with a patient's normal vision and activities.

There is still further a need for a device that can operate reliably while a patient carries on their normal daily activities, and the device does not require a particular critical position or alignment relative to the patient's eyes. The device should be user friendly.

2. Background References. The present application is related to U.S. Patent Application N0. 17/495,198 (Attorney Docket No. 48675-708.301), filed October 6, 2021; U.S. Patent Application No. 17/370.735 (Attorney Docket No. 48675-707.301), filed July 8, 2021; and U.S. Patent Application No. 16/124,630 (Attorney Docket No. 48675-705.201), filed September 7, 2018; and PCT/US2021/15093 (Attorney Docket No. 48675-709.601), filed January 26, 2021.

US 2020/229973 provides a spraying device and a spraying method for moisture mist that supply at least a micro liquid to the eye, and can be used for dry eye symptom relief, drug delivery, allergy prevention, relaxation, and the like. The spraying device comprises a storing part for storing a raw material of a mist-like substance containing the micro liquid and includes a spraying element that sprays a mist-like substance containing the micro liquid onto a local region The raw material is a liquid raw material or a solid raw material that may include a medicinal additive. The medicinal additive is preferably selected from among menthol, analgesics, antibiotics, antiallergic agents, steroids, intraocular pressure-lowering agents, and the like. The spraying device may be an eyeglasses-type spraying device in which the local region is configured by a rim and a temple of eyeglasses, or a cup-type spraying device in which the local region includes an opening.

### BRIEF SUMMARY

The present invention is set out in the appended claims. These and other objectives may be met using the device, system and methods described herein. In various embodiments, the present disclosure relates to an apparatus for delivering a drug to a region near an eye, or on to an eye or a contact lens covering the eye. A system includes the apparatus for drug delivery, and a strain sensor for measuring the intraocular pressure (IOP) of an eye. The present disclosure further includes a method of converting a strain reading from a strain sensor into a proper dose of a drug, to be dispensed from the apparatus for delivering a drug to a region near an eye, on the eye or on a contact lens on the eye. In various embodiments, the IOP may be determined using a contact lens, a camera, and a processor. In some embodiments one or more of these elements may be replaced with an equivalent element. In some embodiments, there may be a drug or medication dispensing device in close proximity to the eye. The device may be a pair of goggles, glasses or other eye wear. In various embodiments, the elements reading the IOP measurement may cooperated with the elements used for drug delivery.

In various embodiments, the present disclosure relates to a drug delivery apparatus for use with a wearable eye wear device. The apparatus comprises a first body defining a fluid reservoir. The fluid reservoir has an open side with a mist generator at least partially covering the open side. A supply tube feeds a volume of fluid into the reservoir, and a fluid sensor detects the presence of fluid in the reservoir. The apparatus may have a second body. The second body has a releasable fastener for engaging a container. The second body may also have a first needle extending into the container, the first needle forming a seal with the container, and able to deliver air into the container. There may be a second needle extending into the container, the second needle forming a seal with the container, the second needle connected to the supply tube. The contents of the container may go through the second needle through the supply tube and into the reservoir. The apparatus also has a pump, wherein the pump delivers air through the first needle and into the container. A controller may determine the volume of fluid in the reservoir based on data from the fluid sensor and wherein the pump delivers air through the first needle and into the container. A controller may determine the volume of fluid in the reservoir based on data from the fluid sensor and cause the pump to activate when the volume of fluid is below a predetermined threshold. A power source provides electricity to the pump, the controller, the fluid sensor, and the mist generator.

In some embodiments, there is a system for the treating an eye. The system comprises a goggle, or other suitable eye wear, positioned in close proximity to the eye. The goggle comprises an optical sensor capable of capturing an image of a strain sensor; a processor that can interrogate or receive data from the optical sensor and determines the amount of strain experienced by the strain sensor. The goggle also has the drug delivery apparatus for dispensing a drug into a volume of space in close proximity to the eye. The drug delivery apparatus has a first body with a mist generator, a supply tube and a fluid sensor; and a second body with a releasable fastener, a first needle and a second needle, a pump, a controller and a power source. The goggle may determine an IOP value based on the data from the optical sensor, and trigger the drug delivery apparatus to dispense a drug.

In some embodiments, there is a system for the treating an eye. The system comprises a goggle, or other suitable eye wear, positioned in close proximity to the eye. The goggle comprises a magnetic sensor capable of determining the position of a magnet or ferro-magnetic material (the magnet being part of a contact lens platform and located on the eye). The goggles may include a processor. The processor may interrogate or receive data from the magnetic sensor and determine a change in position of the magnet, the magnet having a first position and a second position. The goggle may include a drug delivery apparatus for dispensing a drug into a volume of space in close proximity to the eye. The drug delivery apparatus may have a first body with a mist generator, a supply tube and a fluid sensor, and a second body with a releasable fastener, a first needle and a second needle, a pump, a controller and a power source. The processor may determine an IOP value based on the change of position of the magnet between the first and second position. The processor may trigger the drug delivery apparatus to dispense a drug.

There are also described various methods of delivering a drug to an eye. In an embodiment, the method of delivering a medication to an eye involves interrogating, via a processor, a sensor, wherein the sensor contains a data set related to an intraocular pressure of the eye. Then determining, via the processor, the IOP pressure of the eye. Then, comparing, via the processor and a memory device, if the IOP pressure meets a threshold requirement for medication. Then, delivering a medication, via a drug delivery device, into a volume of air in close proximity to the eye. The delivering of medication is performed by a drug delivery apparatus for dispensing a drug into a volume of space in close proximity to the eye; the drug delivery apparatus has a first body with a mist generator, a supply tube and a fluid sensor; the drug delivery apparatus has a second body with a releasable fastener, a first needle and a second needle, a pump, a controller and a power source.

In another embodiment there may be a system for measuring and treating the IOP of a patient. The system may have: a computational device, a wearable eyewear device for collecting IOP data and being in signal communication with the computational device, the eyewear device having a drug dispensing component. The system may also have a database containing a user profile including personalized ophthalmologic reference data where the database may be accessed by the computational device, and where the database, and the IOP data are used to determine a treatment regimen for a user's eye. A drug delivery component on the eyewear may deliver the drug in response to a signal from the computational device.

In various embodiments, the computational device may be a cell phone, a tablet or a laptop computer. In still other embodiments, the computational device may be attached to the wearable eyewear device.

Devices, systems and methods are described herein using eyewear with one or more illuminators and one or more image sensors. The combination of illuminator(s) and image sensor(s) may operate to eliminate one or more of ambient lighting changes and/or misalignment error, while providing a sensitive and accurate measurement of the cornea radius. A small change of the radius of curvature (as small as 4 micrometers per 1 mmHg change in IOP) may be observed for a typical adult cornea. The optical design may allow image processing and sensor fusion, as well as machine learning to accurately and sensitively measure the radius of curvature changes in the cornea. The measured changes may be used in a calculation using a machine learning program, a learning neural network, an artificial intelligence program, or other analytic computational program to relate the measured changes in radius to the IOP. The method may use a preliminary characterization of the corneal thickness and topography where the radius of curvature at a known IOP reading is acquired by conventional ophthalmologic methods. The personalized data set may then use as an input into the data processing algorithms, that also use continuous imaging measurements from the eyewear to calculate the IOP. The data may be connected to a computational device such as a cell phone or the cloud, and the eyewear may dispense a drug using a drug dispensing device. The drug may help reduce the IOP of the eye. The present disclosure includes a wearable optical device that measures the IOP through image acquisition from one or more image sensors, and uses the image data along with a reference data for a particular individual to accurately determine the IOP, and may dispense drugs to the eye to control the IOP.

### BRIEF DESCRIPTION OF THE DRAWINGS

Reference is now made to the drawings in brief, where like part numbers refer to the same part. Otherwise different part numbers, even if similar to other part numbers, represent different parts of different embodiments. Elements in the illustrations are not necessarily shown to scale unless specifically indicated, and may be distorted to some degree to emphasize the element or some characteristic of the element. Not all parts are shown in all embodiments so that the view of the figure does not become unnecessarily distorted.
FIG. 1 illustrates an optical imaging sensor goggle for measuring the intraocular pressure remotely (IOP goggle) according to an embodiment.
FIG. 2 illustrates a top view of an IOP goggle according to an embodiment.
FIGS. 3A and 3B illustrate a goggle according to an embodiment.
FIG. 4 illustrates the change in corneal topography when the IOP changes from 15 to 30 mmHg according to an embodiment.
FIG. 5 illustrates a schematic ray trace showing optical beams bouncing off a cornea according to an embodiment.

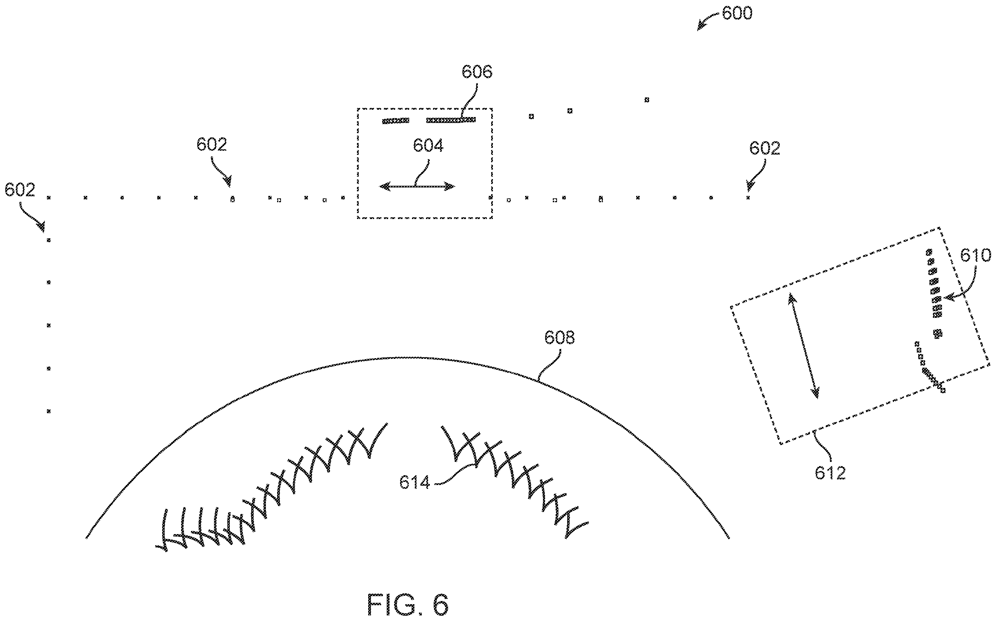

FIG. 6 illustrates a schematic ray trace showing optical beams forming images according to an embodiment.
FIG. 7 illustrates a schematic ray trace that shows images of the point-sources at the camera's image planes when the corneal Radius changes according to an embodiment.
FIG. 8 illustrates a coordinate system used to describe the corneal position according to an embodiment.
FIG. 9 illustrates a schematic ray trace showing corneal X-position changes according to an embodiment.
FIG. 10 illustrates a schematic ray trace showing corneal z-position changes according to an embodiment.
FIG. 11 illustrates a schematic ray trace showing image changes when the corneal angular position changes according to an embodiment.
FIG. 12 illustrates a side facing image sensor and a pattern of light spots according to an embodiment.
FIG. 13 illustrates different light beams intersecting with the cornea according to an embodiment.
FIG. 14 illustrates a calculation showing changes in positions of laser points according to an embodiment.
FIG. 15 illustrates a pattern of light spots on an eye according to an embodiment.
FIG. 16 illustrates a cross section view of two example corneas with different intraocular pressures, according to an embodiment.
FIG. 17 illustrates a graph of data using a polynomial fit according to an embodiment.
FIG. 18 illustrates a schematic of data processing according to an embodiment.
FIG. 19 illustrates a sample logic according to an embodiment.
FIG. 20 illustrates a data processing flow chart according to an embodiment.
FIG. 21 illustrates an example data processing pipeline according to an embodiment.
FIG. 22 illustrates another example data processing pipeline according to an embodiment.
FIG. 23 illustrates a contact lens with IOP measuring capability and a reader device according to an embodiment.
FIG. 24 illustrates an example wearable contact with an IOP strain sensor according to an embodiment.
FIG. 25 illustrates an example wearable contact lens with an IOP strain sensor according to an embodiment.
FIG. 26 illustrates a plan view of a wearable contact lens with an IOP strain sensor according to an embodiment.
FIG. 27 illustrates a set of IOP strain sensor in cross section according to several embodiments.
FIG. 28 illustrates different options for strain sensor set up according to different embodiments.
FIG. 29 illustrates a graph of pressure response to different numbers of rings according to several embodiments.
FIG. 30 illustrates a sensitivity dependence based on the number of reservoir rings according to an embodiment.
FIG. 31 illustrates a cross section view of a contact lens with an IOP sensor placed on the cornea of an eye according to an embodiment.

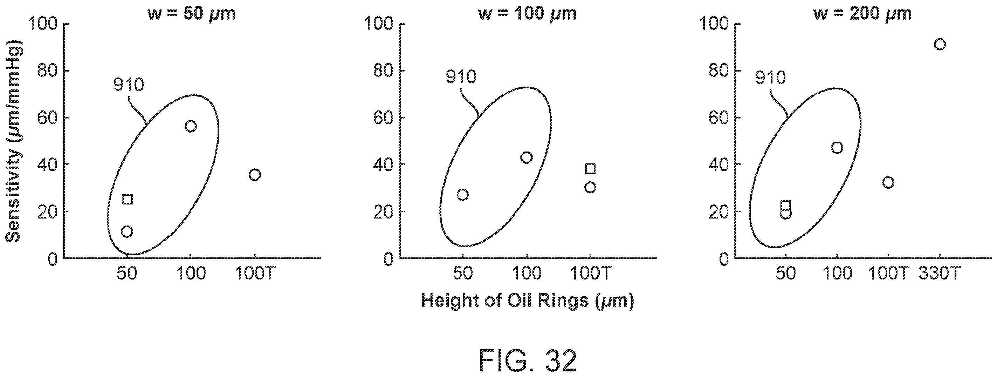

FIG. 32 illustrates sensitivity dependence on the height for three different ring widths in accordance to an embodiment.
FIG. 33 illustrates an auxetic contact lens sensor and close-up view of the liquid reservoir cross section according to an embodiment.
FIG. 34 illustrates a sensor with a reservoir ceiling patterned with circular and linear convex shapes according to an embodiment.
FIG. 35 illustrates a microscope image of the sensor with a linearly patterned liquid reservoir ceiling according to an embodiment.
FIG. 36 illustrates steps that may be used to fabricate the sensor according to an embodiment.
FIG. 37 illustrates steps that may be used to fabricate the sensor according to an embodiment.
FIG. 38 illustrates fabrication steps of a ceiling layer of the auxetic microfluidic sensor according to an embodiment.
FIG. 39 illustrates a strain sensor for biomechanics of cancer cells according to an embodiment.
FIG. 40 illustrates several example shapes of microscopic features according to an embodiment.
FIG. 41 illustrates a graph of COMSOL results of a sample device according to an embodiment.
FIG. 42 illustrates a goggle with an imaging device and a drug delivery system according to an embodiment.
FIG. 43 illustrates an imaging system with a light source according to an embodiment.
FIG. 44 illustrates a side view of a drug dispensing system according to an embodiment.
Fig. 45 illustrates a cross section of a portion of a drug delivery system according to an embodiment.
FIG. 46 illustrates a flow chart of a drug dispensing system logic according to an embodiment.
FIG 47 illustrates an electrical schematic of a goggle with a drug dispensing system according to an embodiment.
FIG. 48 illustrates sample test data of a strain sensor according to an embodiment.

### DETAILED DESCRIPTION OF THE INVENTION

The present disclosure describes wearable eyewear, systems and methods for measuring the cornea of an eye, and determining the intraocular pressure of the measured eye based on the curvature of the cornea. The disclosure includes contact lenses, eyewear, computational devices for calculating IOP values based on cornea data collected by the eyewear. This disclosure also includes methods for calculating the IOP, and dispensing a drug to the eye when needed. Descriptions herein which may use the terms eyewear device or eyewear are meant to be used interchangeably, and reference to either an eyewear device or eyewear is understood to mean any of the wearable eye wear systems, apparatus and devices, as described herein, unless context specifically indicates otherwise.

The eyewear as described herein may take a variety of forms. The form factor may be one of choice for a user, or one for the user's optometrist or other professional medical person responsible for the user's eye health. In some embodiments, the form factor may include a frame and a lens. The frame may be one where the user may wear in front of his eyes (note the use of male or female pronouns may be distributed herein randomly. The disclosed technology is not dependent on the gender of the user. The interchanging use of the gender of the user or other persons described herein is simply for the convenience of the applicant). The frame may be any sort of eyewear frame used for modern eyewear, including frames for sun glasses, vision correction glasses, safety glasses, goggles of all types (e.g. Swimming, athletic, safety, skiing, and so on). The frame may be suitable for a single lens for one eye, a lens for two eyes (e.g. a visor), or a single lens and an eye cover (such as for persons with "lazy eye" or who may suffer from the loss of one eye). The lens may be a prescription lens for vision correction, a clear or tinted lens for appearance, or an opaque lens that covers the eye. In many embodiments, the lens may have a defined area for the field of view of the user. The field of few may be clear to avoid blocking the vision of the user. The various elements of the eyewear device may be place on the periphery of the lens, or on the frame. The frame or lens may have flanges or other protrusions or tabs for the attachment of image sensors, light sources, battery, computational devices, drug delivery devices, or any other component suitable for the use with the present disclosure.

The wearable eyewear may have one or more image sensors positioned to face the eye(s) of the user so the image sensor may capture an image of the eye. The image sensor may be a camera, a CCD (charge coupled device), CMOS (complementary metal oxide semiconductor), or other image capture technology. The wearable eyewear may have one or more light sources for projecting light at the eye. In some embodiments, the light source may be a form of illumination that produces specific wavelengths of light. The light emission may be at a shallow angle to the curvature of the cornea, and projected outside the lens portion of the eye so that the light does not interfere with the users normal vision. In some embodiments the light source may be a laser. In some embodiments the light source may be a LED (light emitting diode), and in other embodiments the light source may be any light generating technology now known or still to be developed.

In some embodiments, the eye wear device may use a magnetic sensor in place of, or in addition to, the image sensor. The magnetic sensor may create a magnetic field. A contact lens platform with a magnet or ferro-magnetic material may be worn on the eye to be examined. The magnetic field may be activated to push the magnet or ferro-magnetic material toward the center of the eye. The magnetic field may be used to evaluate the distance it has been pushed relative to the surface of the eye. The distance depression of the eye surface may be used to determine the IOP value of the eye.

In various embodiments, the light source(s) and image sensor(s) may be positioned so that images captured by the image sensor are able to ignore ambient light, glare or other optical artifacts that might interfere with the accurate reading of the change in cornea curvature. The light source and the image sensor may use one or more polarizing filters to substantially reduce or eliminate light of a particular polarization, wavelength or intensity, so the captured image may have greater reliability and less signal noise. In another embodiment the eyewear may have a light sensor to help regulate when the ambient lighting conditions are appropriate for taking a suitable image of the eye to determine the cornea curvature. The images captured by the image sensors may be stored locally for a period of time, or transmitted to a computational device via a communication portal.

In some embodiments, the communication portal may be an antenna for wireless transmission of data to a computational device. The communication portal may send and receive information, such as sending image data, and receiving dosing information for a drug delivery device. In various embodiments, the computational device may be a cell phone, a tablet computer, a laptop computer, or any other computational device a user may select to carry out program (App) functions for the eyewear device. In some embodiments, the computational device may be resident on the eyewear. In some embodiments, the communication portal may be a wired connection between the image sensors, the light sources, the computational device, and a power supply for all the electrical components. In still other embodiments, the communication portal may connect the eyewear to the cloud.

In an embodiment, there is a method for determining the IOP of an eye. In some embodiments, the method may use a basic operation pipeline. The pipeline may receive image data from a variety of sources. In some embodiments the image data may come from the eyewear as it is worn by a user. In some embodiments the image data may come from a database having stored ophthalmologic data of the user at a fixed point in time. In some embodiments the images may be anatomic data of a user from a fixed point in time. In an embodiment, some or all the available image data may be used in a deep neural network with an image processing front-end. The image processing front-end may derive or calculate an IOP reading. In some embodiments, the IOP reading may be updated at video data rates, providing a quasi-real time output.

In another embodiment, the data pipeline may cause an image sensor to change exposure levels, gain, brightness and contrast in order to capture non-saturated images. The images may be passed through a threshold filter to reduce or eliminate background noise. Some high resolution images may be stored in a temporary memory for rapid processing, while blurry and low resolution images are formed. The low-resolution images may then be passed through a match filter or feature detection filter to pinpoint spots corresponding to particular illumination/light sources in the various captured images. The coarse locations may then be used to segment the high-resolution images and perform peak fitting algorithms to individually determine the positions and widths of each peak in the images. The results of the peak locations and widths may then be used with the previously trained neural network, which may then be used to estimate cornea coordinate and radius of curvature. A nonlinear equation solver may be used to convert the radius of curvature into an IOP reading.

In an embodiment, the IOP reading may then be used to determine a drug dose to administer to the eye being monitored. The drug dose information may be relayed back through the communication portal to the eyewear and the drug dispensing device. The drug dispensing device may then administer the proper dose to the eye. In some embodiments, the drug delivery device may use an atomizer. In other embodiments the drug delivery device may use eye drops. In still other embodiments, the computational device may provide an alert to the user to self-administer a drug of a certain dose at a certain time.

As described herein, a wearable eyewear device may be coupled to a computational device to measure the IOP of a user's eye. The user may be a person wearing the eyewear unless the context of the usage clearly indicates otherwise.

Various aspects, embodiments and examples are described that may be imprecise. In medical technology and treatment, diagnosis, drug prescription and usage, as well as therapy regimens may not be the same for every person do to nuances in individual biology. Thus, various embodiments described herein may use a term such as "generally," or "substantially." These terms should be understood to mean that due to variations of people, and variations of eyes, from each other, and from one person to the next, there may necessarily be variations in how some embodiments operate in calculations, in communications, in data manipulation and in treatment. We refer to "generally" and "substantially" as including any variation.

Reference is made herein to various components and images. The use of the references are to help guide the reader in a further understanding of the present disclosure. In particular, while the singular version of a noun is often used, it should be understood that the embodiments fully consider plural numbers of components and images to also be within the scope of the disclosure.

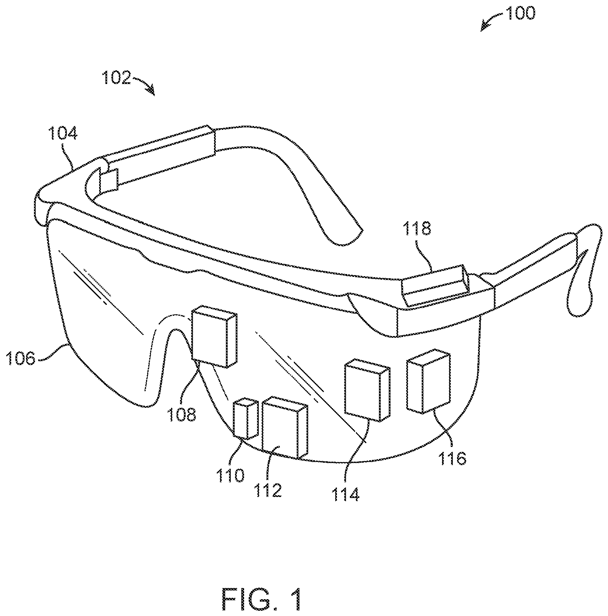

Referring now to the FIG. 1, an eyewear 102 device having a frame 104 and a lens 106 may be provided. The lens 106 may be a single structure as shown, or there may be two lenses as with a pair of glasses. The lens 106 may have a first light source 108, and one or more image sensors 112, 114, 116 placed on it. In other embodiments, any one of the light source 108 or image sensors may be placed on the frame 104. In some embodiments the image sensors and light source 108 may be placed on either the frame 104, the lens 106, or partially on both. The eyewear 102 may also have a drug delivery device 110 positioned to deliver a medication directly to the eye, or to a volume of air in close proximity to the eye. The drug delivery device 110 may be an atomizer or other aerosol device, a dropper or any other device for delivering medication to the eye. In some embodiments, the drug delivery device 110 may be a mist applicator. In some embodiments, the mist applicator may be a MEMS (micro-electro-mechanical systems) atomizer with a drug carrying cartridge. The drug carrying cartridge may be replaced. A controller 118 may control the individual image sensors, the light source 108 and the drug delivery device 110. The controller 118 may be connected to the other components via a wire or cable connection, or by using a short-range wireless communication protocol to each. In some embodiments, each component may have its own power source. In some embodiments, a single power source may be wired to each of the components to power all the components as needed. In some embodiments, a combination of power sources, local and central, may be used.

In various embodiments, the power supply to the controller and other components may be replaceable. In some embodiments, there may be a drug reservoir (not shown) associated with the drug delivery device 110, and the drug reservoir may be replaceable, or refillable. In some embodiments, the drug reservoir may be a drug cartridge. In some embodiments, the drug reservoir may be a chamber or other container that may receive a drug or medication from a storage device, such as a cartridge. In the drawing, the components are depicted as simple shapes for illustration purposes only. The components are not to scale on the eyewear 102 and no interpretation of the size of the components should be assigned to them based on the drawing figure. The location of each component may also vary from one embodiment to the next, and the location presented is merely illustrative. The drawing figures are for illustration purposes only.

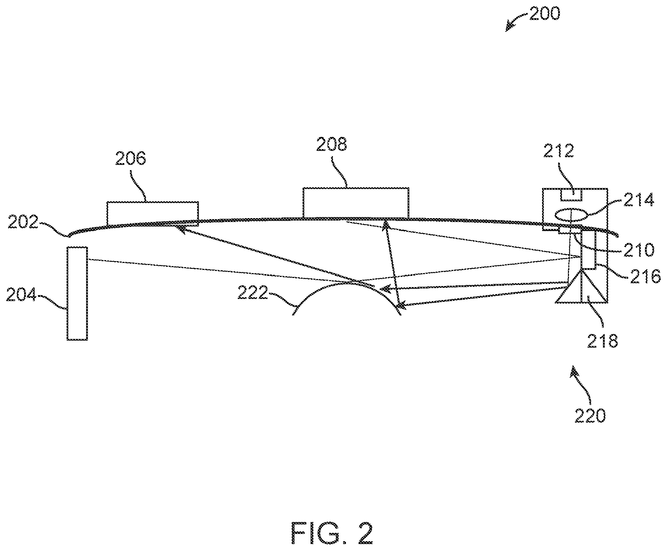

In an embodiment, there may be an optical design for the eyewear 202 as shown in FIG. 2. The eyewear 202 may be fitted with a side illuminator made up of a planar illuminator 216, a laser diode 212 collimated by a collimator lens 214 and multiplied into a pattern by a hologram 210. The assembly of the planar illuminator 216, laser diode 212 and collimator lens 214 may make up a light source 220. The hologram 210 may be relayed towards the cornea 222 by a mirror 218. The reflections of the hologram 210 off the cornea 222 may be captured by one or more image sensor 204, 206, 208. In an embodiment, the planar illuminator 216 may provide wide angle and uniform illumination, allowing the image sensors to acquire images of the eye. The planar illuminator 216 may be turned on to acquire a background image of the cornea, pupil, and iris. It may then be turned off to allow background free image collection from other light sources such a laser diode 212, or any other light source that may be provided.

In an embodiment, the laser diode 212 may project a laser beam through the collimator lens 214 and through a hologram 210. The hologram 210 image reflects off the mirror 218 and shines on to the cornea 222. Depending on the curvature of the eye, the hologram image reflects to a first image sensor 208 and a second image sensor 206 as shown by the arrows. In this embodiment, the side image sensor 204 does not capture any image from the hologram reflection of the cornea 222. The various image sensors may capture images and send the image data to a processor. The processor may be on board the eyewear device, or the processor may be remotely located, as a cloud processor, or a processor that may be linked to the eyewear device, such as a smart phone, tablet, or laptop computer.

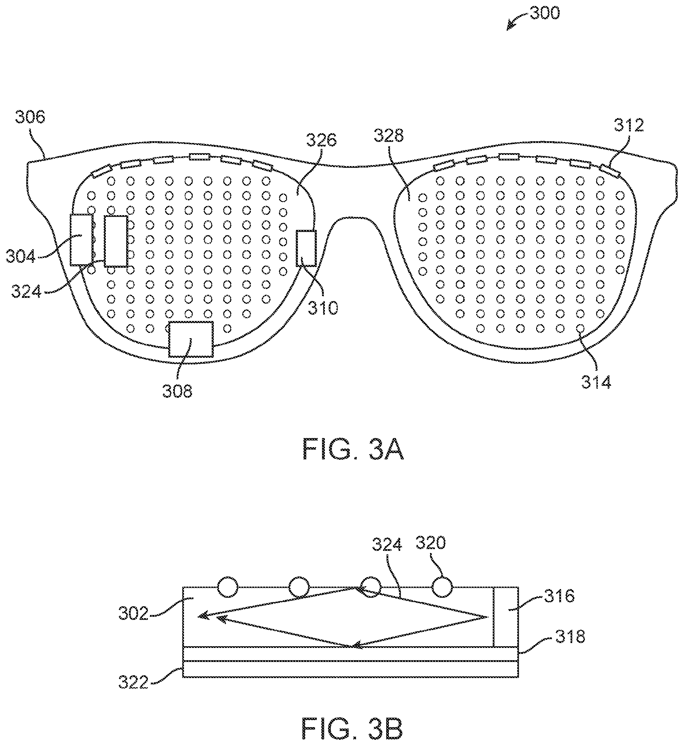

In an embodiment, an eyewear device 302 may be provided as shown in FIG. 3A. In an embodiment, the eyewear device 302 may have a frame 306 holding a first lens 326 and a second lens 328. Image sensor 304, 308, 324 may be attached to the inside (facing the eye) of the lenses or the frame. A light source 310 may be positioned near the nose bridge of the eyewear frame 306. The positions of the light source, image sensors and other components may be changed to suite variations in design or patient needs.

In an embodiment, a cross section of a lens 326, 328 is shown in Fig. 3B. The eyewear lens has an array of spherical defects, which may also be illuminators 320 positioned in a variety of different patterns and densities. A side illuminator 316 may project line into the lens. A low refractive index cladding layer 318 and a linear polarization film 322 form the front layer of the lens. The light 324 from the side illuminator 316 travels through the lens.

In operation, the eyewear according to an embodiment may be fitted with planar side illuminator 312, 316, as well as an array of illuminators 314, 320 that may be embedded into the front cover of the lens of the eyewear 302. A linear polarization film 322 may allow one (vertical, horizontal or other planar orientation) polarization from the ambient light into the eyewear device 302 to facilitate vision while blocking the other polarization. This relationship may help the eyewear device to work without interference of any ambient light at the linear polarization film 322. An eye facing image sensor 308, secondary image sensor 324 and a sideview image sensor 304 may have a crossed polarizer that may block the ambient light admitted by the linear polarization film 322. The various image sensors may have pre-established positions relative to an eye. A program that may evaluate data from an image sensor may take into account the position of the image sensor relative to the eye, in order to determine accurate readings from the image data. In some embodiments, each image sensor may have a different calculation depending on its relative position to an eye, a light source, the lens, the eyewear device or any other object or fiducial used by the present disclosure.

In some embodiments, a drug delivery device may be incorporated into the eyewear to dispense drugs for IOP control based on the IOP readings. A waveguide approach to generating a see-through illumination pattern may be seen in the diamond shaped arrows in the cross section image of the lens. The windows of the eyewear have an array of spherical defect 314 and may be illuminated by a side illuminator 316 from within the lens. The lens may be coated with a low refractive index cladding layer 318 to separate the waveguide from the linear polarization film 322.

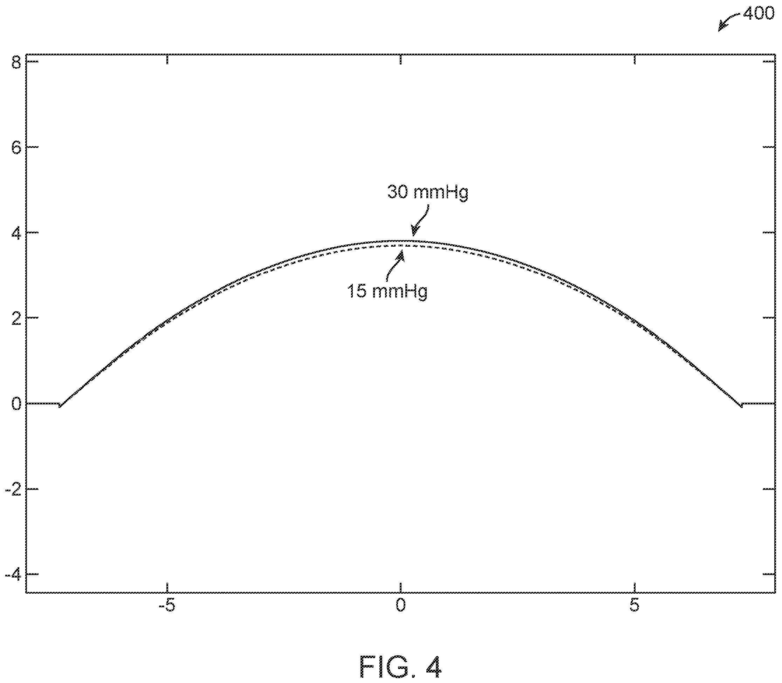

An illustration of the cross section of corneal deflection is shown in FIG. 4. The illustration shows two curves, one raised slightly above the other. The top curve illustrates the corneal displacement of 30 mm Hg (30 mm of mercury pressure) and the bottom curve shows the corneal displacement for half that pressure, or 15 mm HG. The illustration provides two examples where the radius and the apex of the cornea may change due to IOP within the eye.

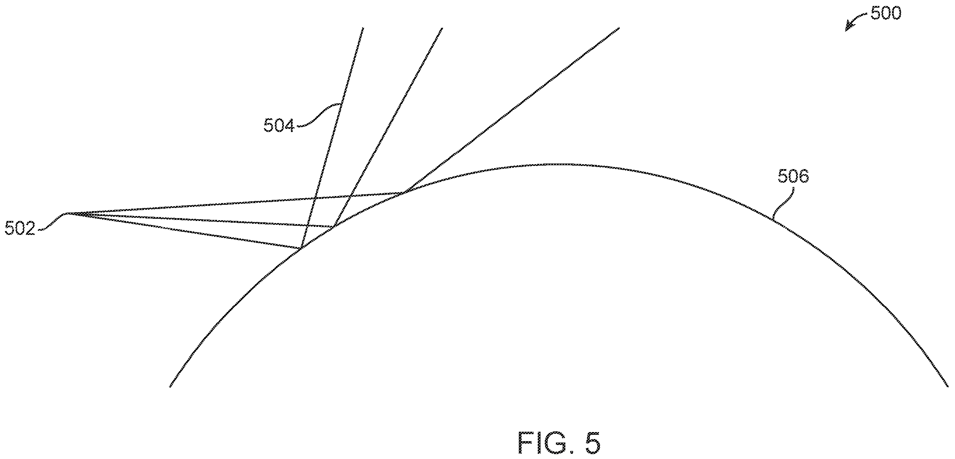

An example of a ray trace diagram is now shown in FIG. 5. In an embodiment, a point source 502 may project light on to the surface of the cornea 506. The light rays may be reflected off the cornea 506 and form one or more reflection 504. The curvature of the cornea 506 as well as the angle of incidence and angle of reflection may be determined using the known position of the point source 502 relative to the cornea, the known angle of image capture by one or more image sensors, and the dispersion of the light from the point source as seen in the images captured.

In an embodiment, an example ray trace from multiple point sources 602 lighting may be arranged around the cornea 608. The light from each of the many point source 602 lighting may be captured at image sensor 604 and image sensor 612, producing real image 606 and real image 610 respectively. Virtual images 614 may also be conceptualized.

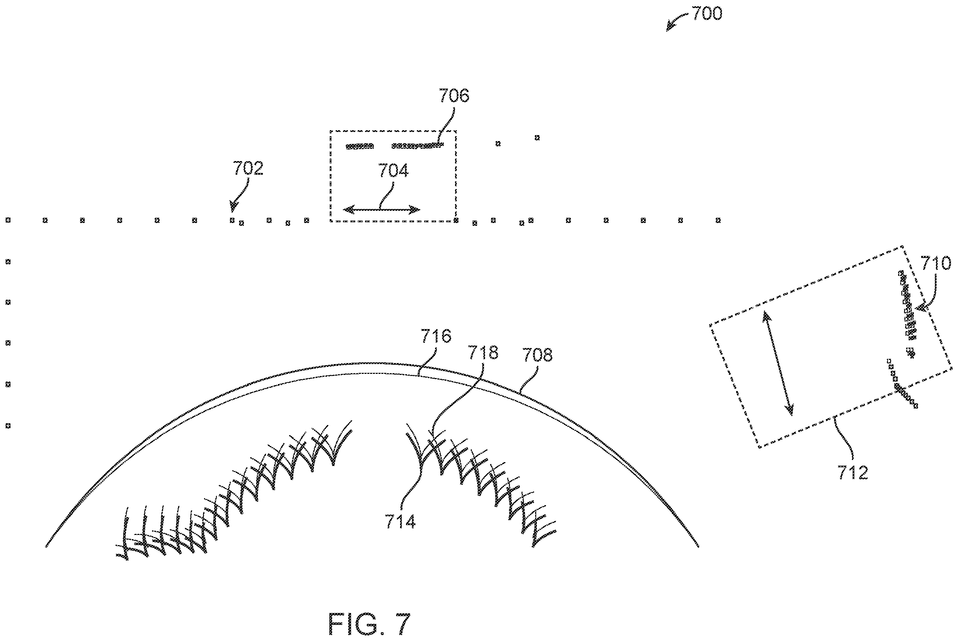

In another embodiment, an example ray trace illustration for two different cornea radii are shown in FIG. 7. The two example corneal IOP pressures are 15 and 30 mmHG. As previously described, a series of multiple point sources 702 may be arranged around the cornea. A front image sensor 704 and a side image sensor 712 may be positioned to capture real image 706 and real image 710 respectively. In various embodiments, light from the multiple point sources 702 bounces of the cornea and the reflected light may be captured by the image sensors 704, 712. In the case of a low pressure cornea, the 15 mmHg cornea 716 has a lower y-axis projection, or a larger radius of curvature. The 30 mmHg cornea 708 has a higher y-axis projection and a smaller radius of curvature. The two cornea pressures may also cause the creation of two different virtual images, a 15 mmHg virtual image 714 and a 30 mmHg virtual image 718. The virtual cornea images may be formed below the surface of the cornea. The positions of the spots corresponding to the multiple point sources in the real images may be different for the two IOP values (15 and 30 mmHg), demonstrating the possibility of using such images to calculate IOP values for the eye.

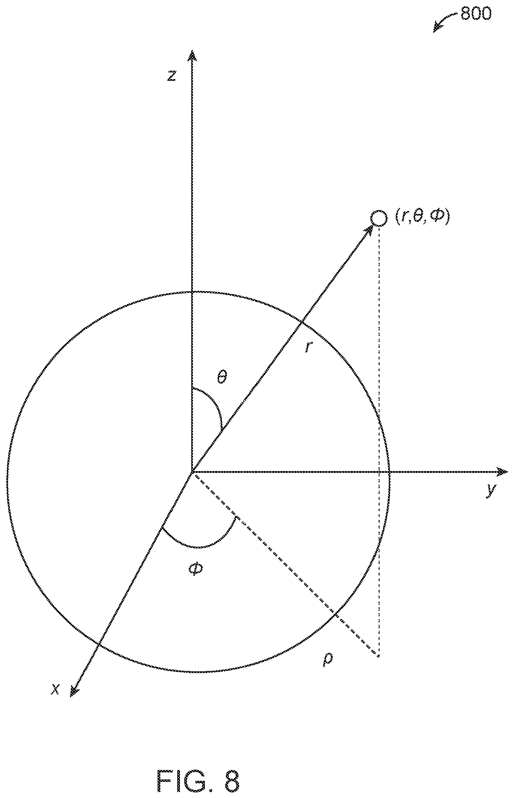

An example coordinate system is shown in FIG. 8. The origin of the spherical coordinate system may be the center of vision for the eye, or an arbitrary position along the cornea or inside the eye. Note that in the various embodiments, the orientation of the x-Axis does not reduce generality.

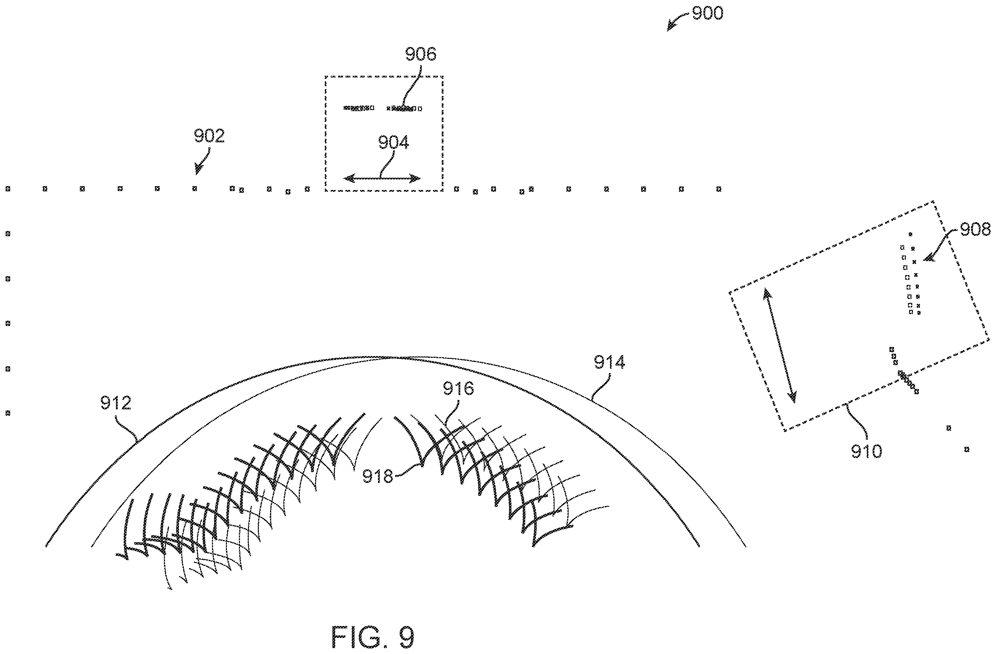

In an embodiment, the shifting of the cornea in a direction may be detected as shown in FIG. 9. In an embodiment, the multiple point sources 902 are arrayed around the cornea. A first image sensor 904 may capture a first real image 906, while a second image sensor 910 may capture a second real image 908. Second real image 908 may vary from one image to another based on the x-axis shift of the cornea over time. A left shift cornea 912 may be slightly shifted from the position of a right shift cornea 914, with corresponding left shift virtual image 918 and right shift virtual image 916 respectively. Using the shifted images between a first point in time T1 and a second point in time T2, the shift in the cornea may be imaged, and used to determine the shift in the X-position of the cornea. Image analysis may be used to correlate the image data to produce reliable x-shift information.

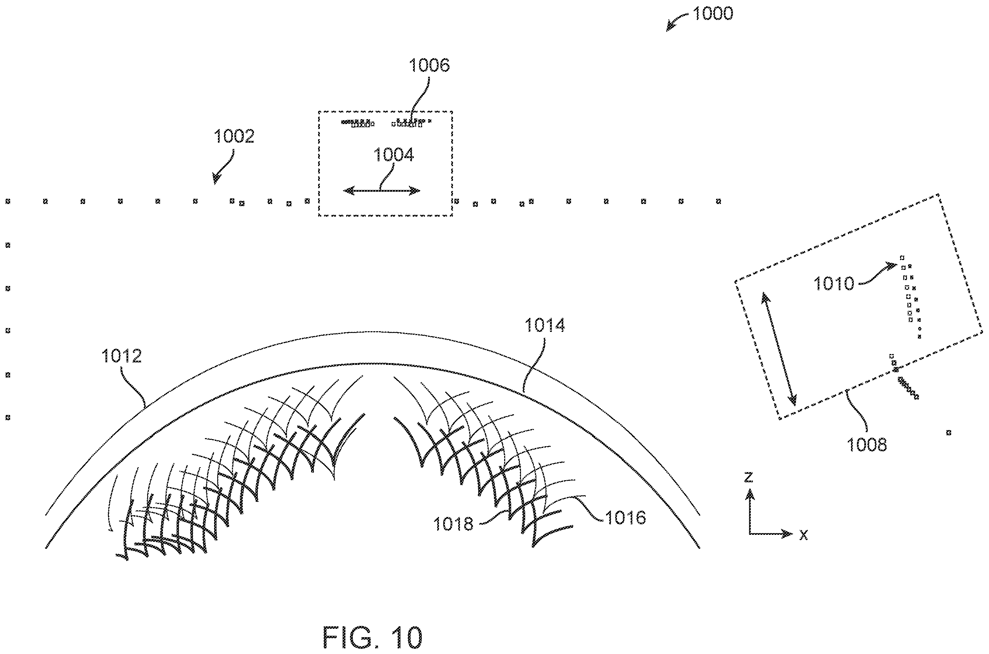

In another embodiment, the z position shift of the cornea may be determined as shown in a ray trace illustration as shown in FIG. 10. In an embodiment, multiple point sources 1002 produce light that reflects off the cornea. The reflected light may be captured by image sensor 1004 and side image sensor 1008. Real image 1006 and real image 1010 are collected from the image sensors. The cornea of the eye may shift in a z axis direction. In some embodiments, there may be z shift positive 1012 and a z shift negative 1014 corresponding to the movement of the cornea. Virtual images may be similarly adjusted, producing a positive virtual image 1016 that may correspond to the z shift positive 1012 and a negative virtual image 1018 that may correspond to the z shift negative 1014 cornea position. The positions of the spots in the real images 1006, 1010 represent different Z positions. The difference may be used to extract the Z position of the cornea through analysis of one or more of the various images.

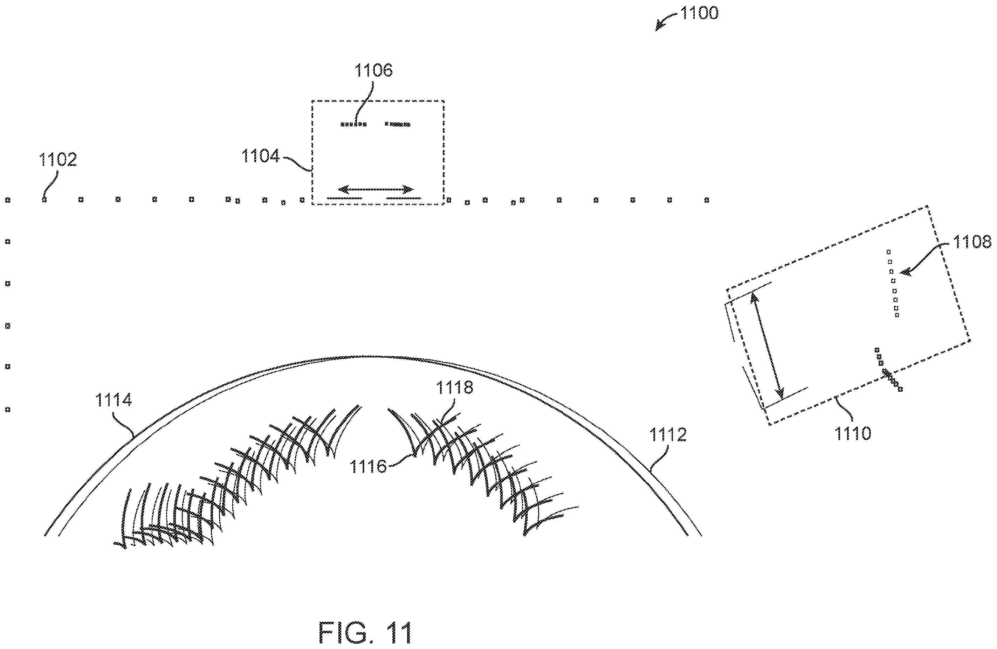

In another embodiment, the angular (theta) shift may be determined using ray trace images as shown in FIG. 11. In an embodiment there may be multiple point sources 1102 of light. The light may reflect of the cornea and images may be captured in a front image sensor 1104 and side image sensor 1110, each producing real image 1106 and real image 1108 respectively. The cornea theta positive 1112 may represent a positive shift in the theta direction, while a cornea theta negative 1114 may represent a negative theta position shift. A positive virtual image 1116 and negative virtual image 1118 may also be detected. The positions of the spots in real images 1106, 1108 may represent two different theta tilt positions. The difference may be used to extract the angular tilt theta of the cornea through the analysis of the images 1106, 1108.

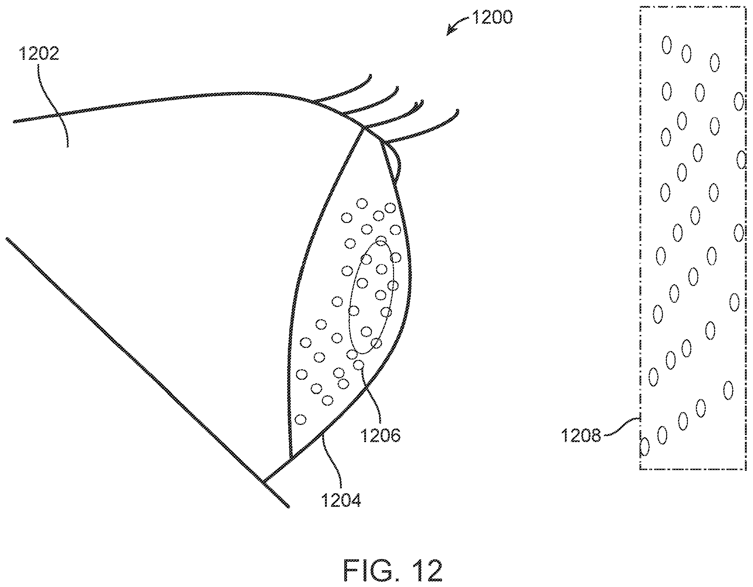

In an embodiment, a side view image of an eye 1202 may be seen, captured through a side facing image sensor (not shown), while the eye may be illuminated using a matrix pattern 1208 from the front, as shown in FIG. 12. In an embodiment, the cornea 1204 may reflect a pattern of illumination 1206 caused by the matrix pattern 1208. In some embodiments, the illumination source may produce a pattern of dark spots, which may be used instead of illumination spots or patterns.

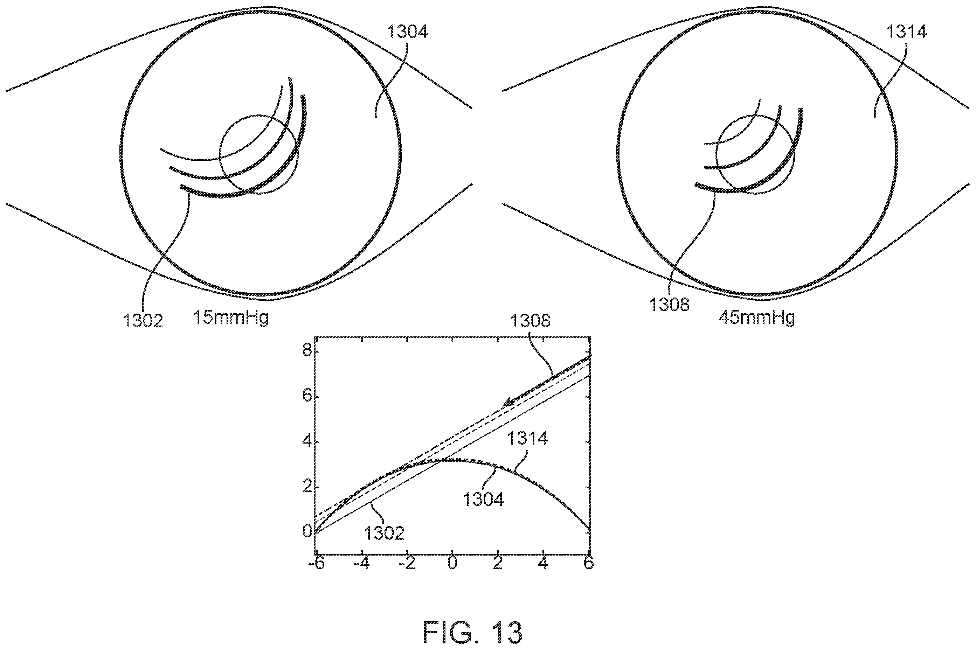

In an embodiment, there is shown another example of illumination using laser energy formed into lines as shown in FIG 13. In an embodiment, there may be shown a computation for light lines incident on the cornea under two different pressure levels. It may be seen that the lines intercept the cornea at different positions for different IOP values. When the crescent shaped curve images are analyzed along with images of the point sources, the images contain enough information to accurately estimate the eye position with respect to the eyewear position, the corneal radius and the IOP.

According to an embodiment, an eye with a lower IOP 1304 and a second eye with a higher IOP 1314 may have a light source illuminate a cross section of the cornea at a given height, or distance from some aspect of the eye. In an embodiment, the lower IOP cornea 1304 may be illuminated with a light source producing a first arc 1302. A second portion of the cornea 1314 may be illuminated when the IOP value of the eye may be higher, and produce a second arc 1308, corresponding to a second light source illuminating the eye at a different height from the first arc.

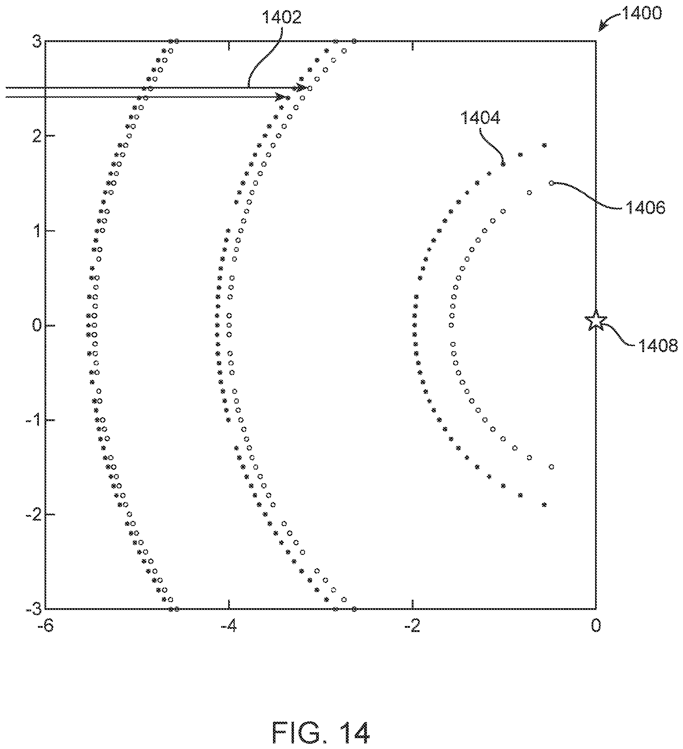

In another embodiment, the intercept positions of a multitude of laser energy may be formed into spots by the hologram and may be calculated for two different IOP values as shown in FIG. 14.

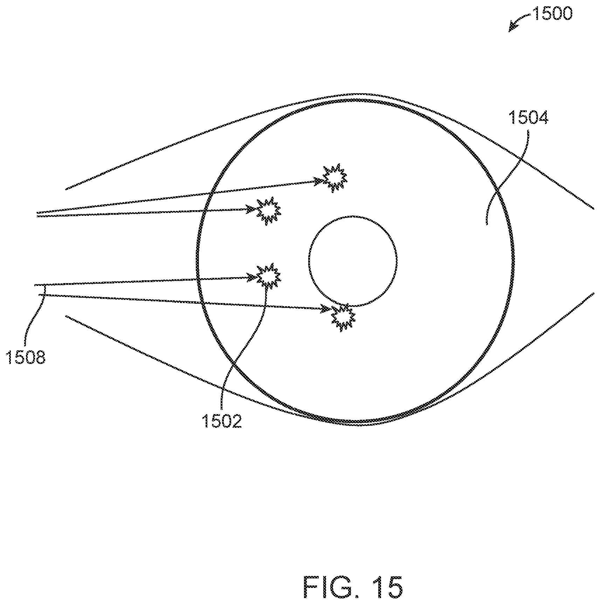

In another embodiment, a video frame capture from an eye facing image sensor with multiple light spots as shown in FIG 15. The reflection of the illuminating spots from the cornea, similar to the positions of spots previously described, may be visible by an eye facing image sensor (not shown). The incoming light 1508 may be coherent light, monochromatic light or other pinpoint light sources which strike the cornea 1504 at specific locations 1502. The reflected light from the various locations 1502 may be captured by the image sensor facing the eye.

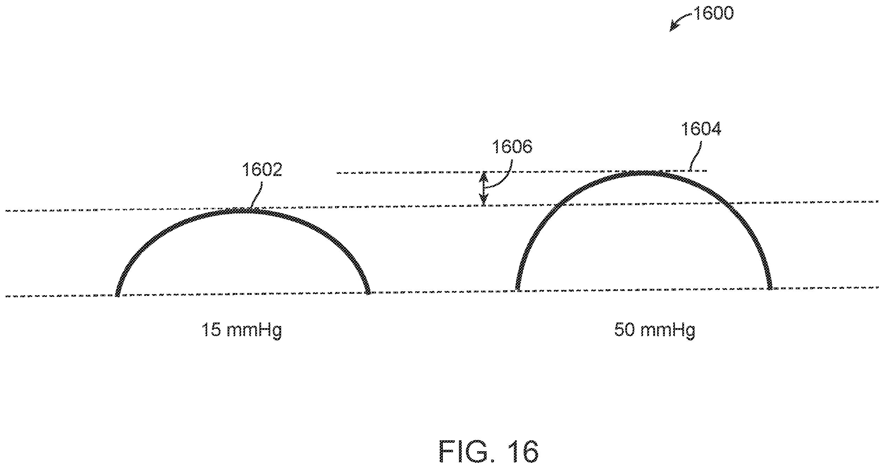

In another embodiment, a side view of a model cornea under two different pressure settings may be seen in FIG. 16. The left figure represents the curvature and bulge of the cornea model when the model is exposed to about 15 mmHg of fluid pressure. The right figure shows a slight increase in the bulge of the model when exposed to about 50 mmHg pressure. The curvature of the model cornea may also be changing as the pressure increases or decreases. The curvature and bulge may be measured using the various techniques described herein. In an embodiment, the lower pressure IOP value may be represented by a forward or upward extension limit 1602, while under the higher IOP value, the eyeball or cornea may extend to a second upward extension limit 1604. The difference between the two extensions may be determined to be a set height difference 1606.

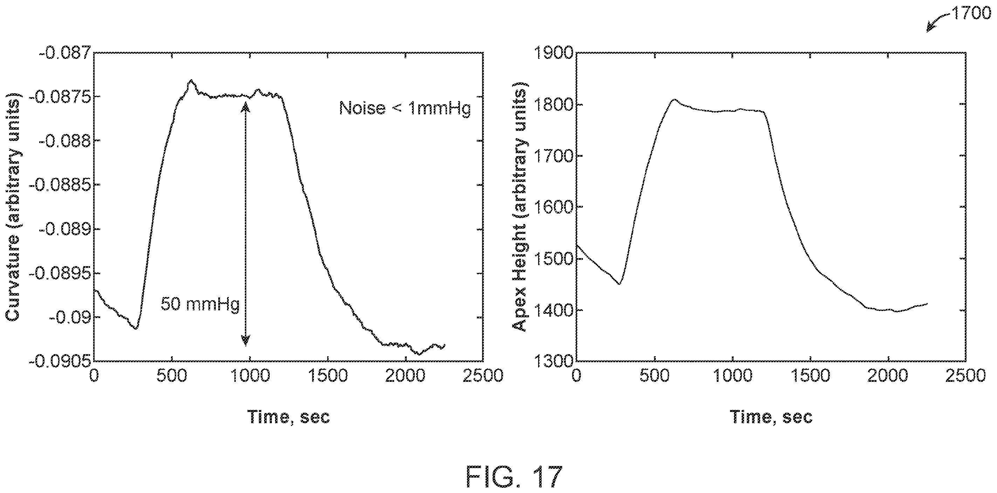

In an embodiment, the curvature of the cornea may be captured in images, and quantified through analysis as shown in FIG. 17. The images may be processed to extract the interface between the cornea and air and to perform a polynomial fit to the extracted curves. The curvature and peak position may be separately extracted and plotted as shown in the top left and right plots. The changes in applied pressure may be accurately extracted from the fitted curves with a noise level below about 1 mmHg. In various embodiments, the fits may be high order polynomials - allowing baseline shifts due to linear positional shifts to be reduced or eliminated. In various embodiments, the image data from the image sensors on the eyewear may be input to a deep neural network that may be composed of image processing components, to reduce the image data to a set of data points. The image processing pipeline may contain trained feature extractors or matched-filtering, edge detection algorithms, filtering algorithms and/or other filters and algorithms. The use of several image sensors may allow determination of the position of the eye with respect to the illumination and eyewear image sensors as well as the head of the user. The algorithms may then be used with neural networks and conventional mathematical fitting methods to extract with high precision the curvature of the cornea.

The curvature of the cornea and height of its apex are plotted in arbitrary units in Fig 17.

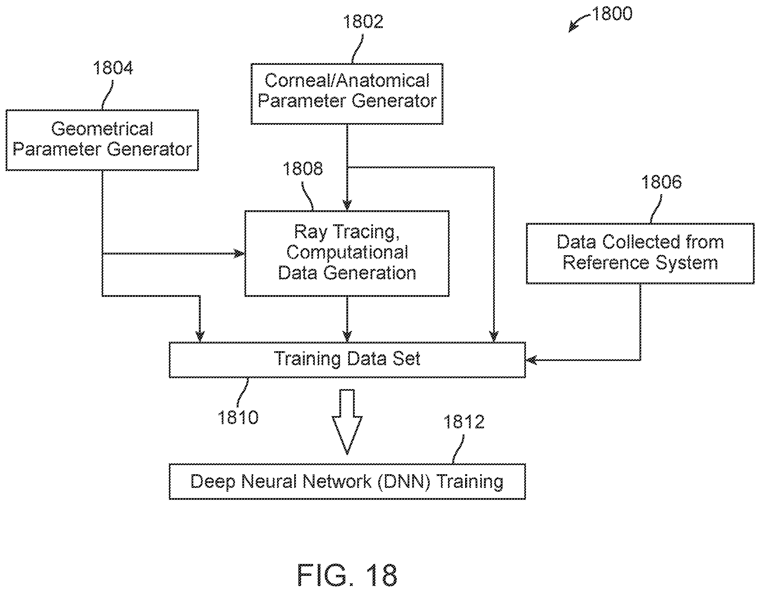

In an embodiment, there may be a method of training a neural network or deep neural network, as shown in FIG. 18. In an embodiment, the schematic diagram of the training method for the neural network/deep neural network (NN/DNN) may involve having the user undergo standard ophthalmologic measurements. These measurements may give accurate values for personal values of cornea thickness, position, and corneal topography in relation to a reference IOP level. The user may also undergo a brief data collection process where the eyewear may be used and reference data may be collected 1806 at the given IOP. In this fashion the eyewear may be calibrated to an individual user. All of this may be data collected from a reference system or systems. These measurements may personalize the system for a user with a unique personal corneal topography. The data collected from personalized measurements may then be fed, along with a computational model ("Geometrical Parameter generator" 1804 and "Cornel/Anatomical parameter generator" 1802), into a ray tracing system 1808 to generate large amounts of image data for a wide variety of parameters 1810. The outputs may then be used with the NN/DNN that contains an image processing pipeline to estimate corneal Radius and IOP 1812.

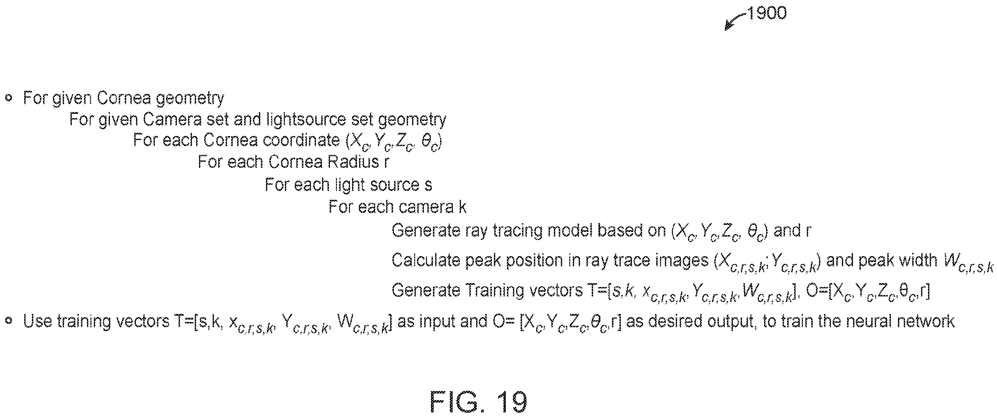

In an embodiment, there may be an algorithm for the generation of training data sets for the training of a neural network, or a deep neural network, as shown in FIG. 19. The locations of spots in the real images from the various image sensors may be calculated for a variety of cornea positions and tilts, as well as cornea radii using ray tracing simulations. The locations of the spots and widths of the spots may be extracted from the ray tracing simulations and may form into vectors to be input into the neural network training software, and original cornea positions may be fed as desired outputs. The training procedure with a large data set may permit the neural network to handle this highly nonlinear problem to be solved with sufficient speed and accuracy. In an embodiment, the material shown in FIG. 19. May be considered as "pseudo-code" that summarizes the steps of data generation from ray-tracing simulations and formatting of the data to train the neural network.

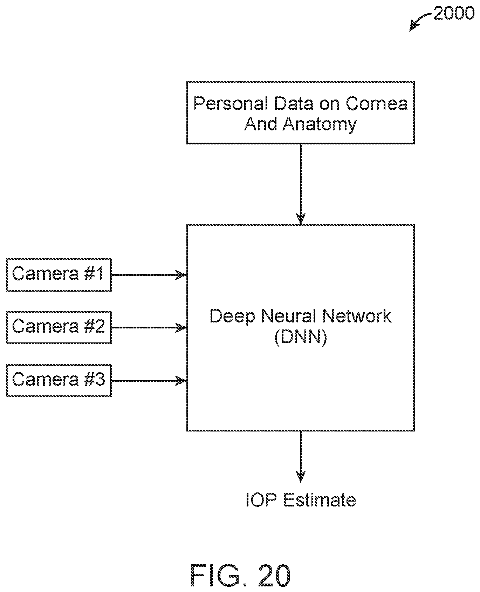

In an embodiment, the basic operation pipeline of the eyewear during measurement may be seen in FIG. 20. The eyewear may use image sensors, such as cameras, to capture images. The captured images may be combined with the personal ophthalmologic and anatomic data. The images may be fed into the deep neural network (DNN) with an image processing front-end, to achieve an IOP estimate. The IOP estimates may be updated at video rates, providing near real time output.

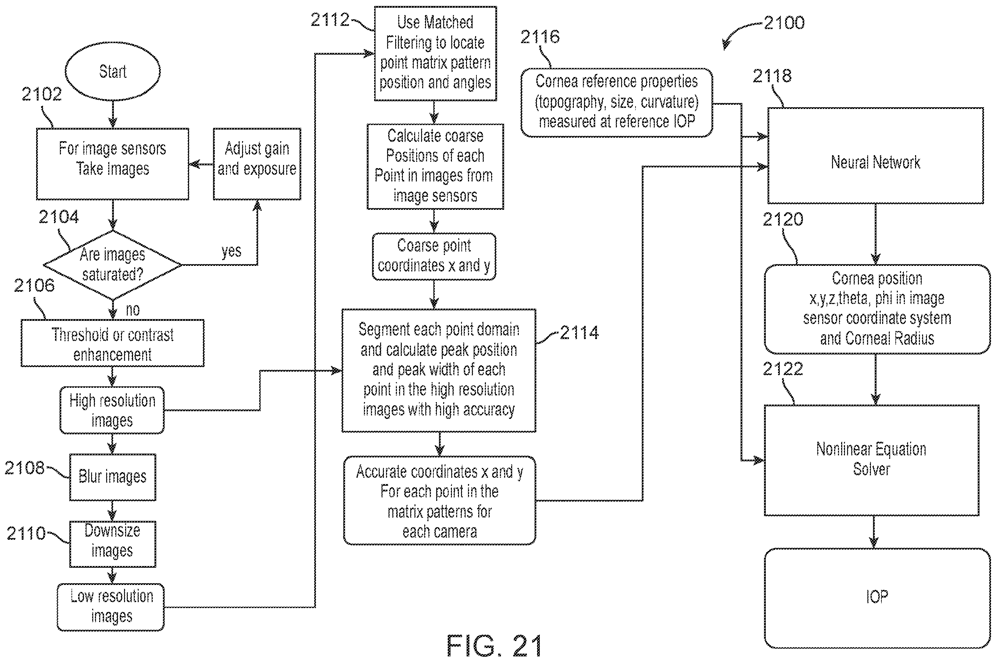

In an embodiment, the pipeline for data processing 2100 may be seen in greater detail, as may be seen in FIG. 21. In an embodiment, the exposure level, gain, brightness and contrast settings of the image sensor may be adjusted rapidly to capture 2102 non-saturated images. In some embodiments this adjustment may be done for each light source, even if there may be multiple point sources as described herein. The images may be evaluated 2104 for image saturation, and if the image saturation is too high, the gain and exposure of the image sensor may be adjusted, and the image taken again. If the image saturation is acceptable, the images may be passed through a threshold filter 2106, eliminating the non-relevant background signals. High resolution images may be stored in a temporary memory. The high-resolution images may be used to create blurred 2108 and lower resolution images 2110 (which may be useful for faster processing). The low-resolution images may then be passed through a match-filter 2112 or feature detection filter to locate point matric pattern position and angles. This function may allow the filter to identify each pinpoint of light in the image and match that pinpoint of light to the corresponding multiple point sources of light in each of the real images. The process may then calculate the coarse positions 2114 of each point of light in the real images from the image sensors. The process may then produce the appropriate x and y coordinates for each real image. The coarse locations may then be used to segment each point domain and calculate peak position and peak width of each point in the high resolution real images with accuracy. The accurate coordinates of x and y positions for each point in the matrix pattern for each image sensor (camera), as well as width of peaks may then be produced. The coordinate data, along with the cornea reference properties 2116 may then be fed into the neural network or deep neural network. The cornea reference properties may include, by way of nonlimiting examples, the topography of the cornea, the size, the curvature, and any other measurement taken at the reference IOP). The results of the peak locations and widths, and/or the accurate measurements, may be used with the previously trained neural network/DNN 2118 to estimate 2120 cornea position x, y, x, theta and phi in image sensor coordinate system and corneal radius (radius of curvature). A nonlinear equation 2122 solver may be used to convert the radius of curvature into an IOP reading.

In another embodiment, the IOP reading may be used with a lookup table (not shown) to determine a dose of a drug. The drug dose may then be dispensed through the drug delivery device.

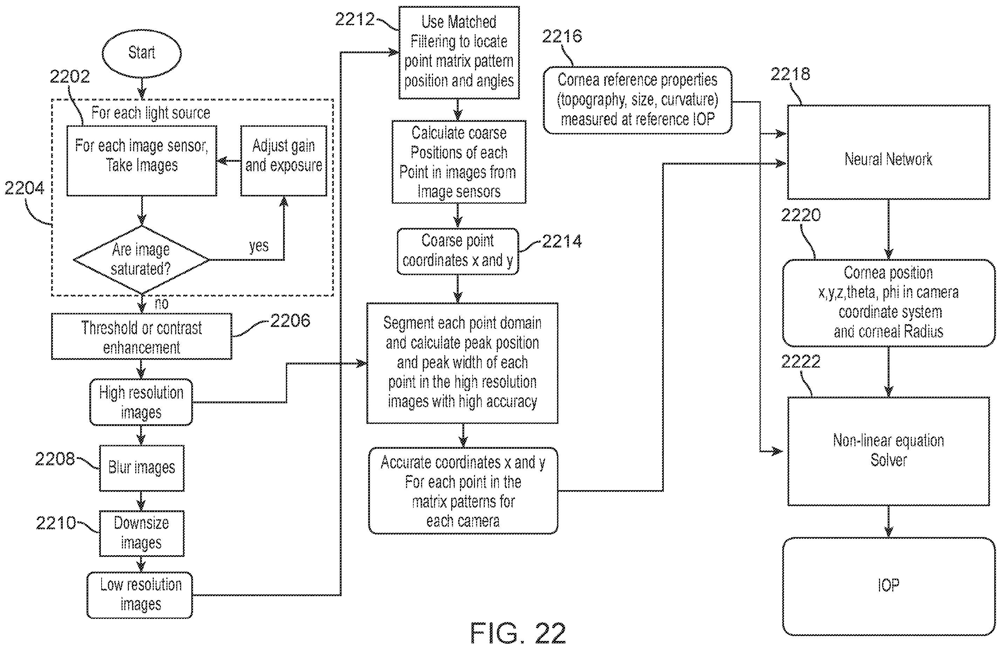

In another embodiment, the pipeline for data processing may be adjusted to include a switching between different illumination sources at the beginning of the pipeline as shown in FIG. 22. The switching between different illumination sources may allow facile separation of image spots in the real images corresponding to different light sources, thereby speeding up the image processing, as well as potentially improving accuracy of data collection. In an embodiment, the exposure level, gain, brightness and contrast settings of the image sensor may be adjusted rapidly to capture 2202 one or more non-saturated images. In some embodiments this adjustment may be done for each light source for each exposure or image, even if there may be multiple point sources as described herein. The images may be evaluated 2204 for image saturation. In some cases, the image saturation may be too high, in which case the gain and exposure of the image sensor may be adjusted, and the image taken again. If the image saturation is within acceptable limits, the images may be passed through a threshold filter 2206, eliminating the non-relevant background signals. High resolution images may be stored in a temporary memory. The high resolution images may be used to create one or more blurred 2208 and/or lower resolution images 2210 (which may be useful for faster processing). The low-resolution images may then be passed through a match-filter 2212 or feature detection filter to locate point matric pattern positions and angles. This function may allow the filter to identify each pinpoint of light in the image and match that pinpoint of light to the corresponding multiple point sources of light in each of the real images. The process may then calculate the coarse positions 2214 of each point of light in the real images from the image sensors. The process may then produce the appropriate x and y coordinates for each real image. The coarse locations may then be used to segment each point domain and calculate peak position and peak width of each point in the high-resolution real images with accuracy. The accurate coordinates of x and y positions for each point in the matrix pattern for each image sensor (camera), as well as width of peaks may then be produced. The coordinate data, along with the cornea reference properties 2216 may then be fed into the neural network or deep neural network. The cornea reference properties may include, by way of nonlimiting examples, the topography of the cornea, the size, the curvature, and any other measurement taken at the reference IOP. The results of the peak locations and widths, and/or the accurate measurements, may be used with the previously trained neural network/DNN 2218 to estimate 2220 cornea position x, y, x, theta (θ) and phi (φ) in image sensor coordinate system and corneal radius (radius of curvature). A nonlinear equation 2222 solver may be used to convert the radius of curvature into an IOP reading.

In various embodiments, the virtual images generally may not be used themselves in the process. The real images may be formed from the virtual images after the image sensor focus light from the virtual images onto the imaging plane of the various image sensors.

The advantages of the present disclosure include, without limitation, a robust process for making of highly sensitive wearable contact lens sensors that have no electrical power or circuits and may be monitored remotely by a simple camera like one found in a mobile phone.

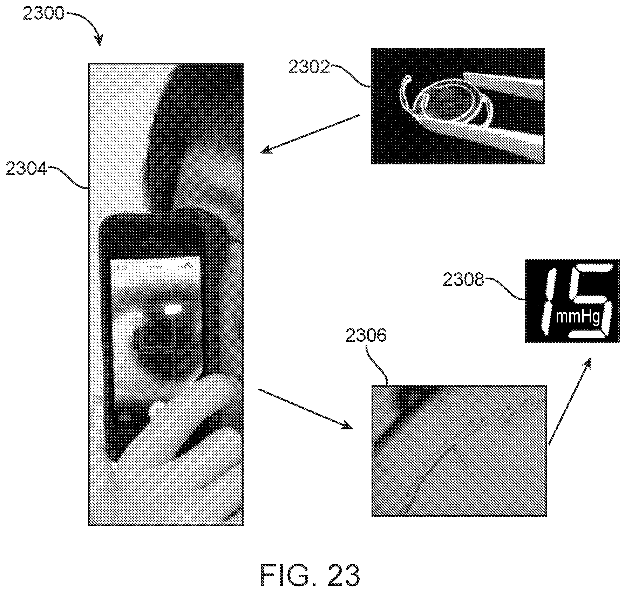

FIG. 23 shows an example of a workflow of the IOP self-measurement technique 2300 according to an embodiment. In an embodiment, a contact lens may be distinct from other available sensors because patients may be able to place and remove the contact lens by themselves. The IOP sensor contact lens 2302 may be similar to a regular contact lens from a use perspective. As IOP fluctuates, radius of corneal curvature may change. In one non-limiting example, a 1 mmHg change in IOP may cause about 4 mm change in radius of curvature. In an embodiment, the fluidic level in the microfluidic sensing channel 2306 of the sensor may change as a response to radius of curvature variations on the cornea. The sensor response may be detected with a smartphone camera 2304 equipped with an optical adaptor and then converted to pressure value by a smartphone app 2308. In some embodiments, a wearable eye sensor as described herein may be used. While the image shows a microfluidic strain sensor, the strain sensor may be any suitable for use on a contact lens. Such strain sensors may include, but are not limited to, a distortion sensor using visual fiducials, which may be measured by a camera and algorithm that determine the strain based on the distortion of the fiducials, or the distortion of the pattern of fiducials. Other strain sensors which may rely on optical reading, magnetic reading or any form of electromagnetic reading, may also be possible. In some embodiments the strain sensor may be replaced with a magnetic field sensor. The magnetic field sensor may be used with a contact lens having a magnet or ferro-magnetic material instead of a strain sensor.

In an embodiment, a patient may wear a contact lens 2302 (Fig. 23), which may be read using an optical sensor, such as a cell phone camera 2304. The camera 2304 provides the image data of the IOP reading to a processor 2306, which may use the data to evaluate the IOP of the eye. In some embodiments the optical sensor may be part of an eye wear device, apparatus or system.

In some embodiments, microfluidic circuits, analogous to electronic circuits, may function as low or high pass filters (electrical resistance and capacitance may be replaced by fluidic resistance (R) and the compliance (C) of compressible materials, respectively). The RC value may determine the time constant of the sensor response. Sensors with large RC values may not respond to fast changes but may be sensitive to slowly varying diurnal variations. Sensors with small RC values may have the capability to detect the effects of blinking and ocular pulsation.

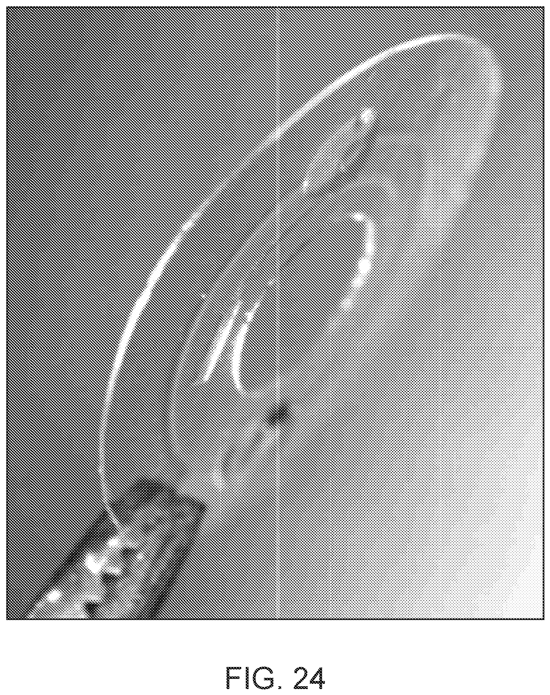

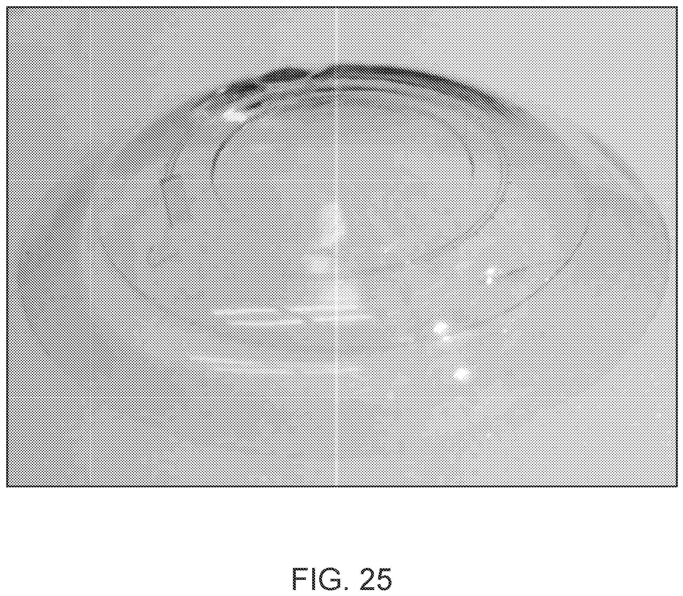

In an embodiment, the microfluidic strain sensor (FIG. 24) may be integrated into a contact lens (FIG. 25) for wearable sensing applications. In an embodiment, the sensor and contact lens platform may be 1 mm thick or less. In some embodiments the sensor and contact lens may be less than 500 microns thick. In still other embodiments, the sensor and contact lens platform may be about than 300 microns.

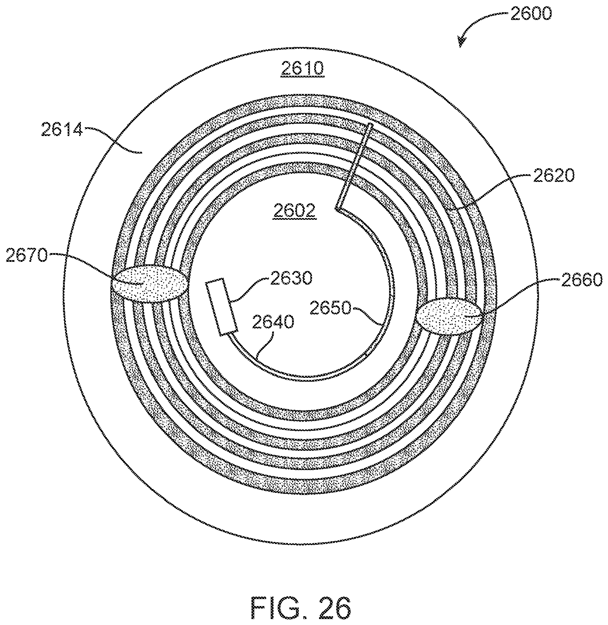

In an embodiment, a top view of a closed system sensor with multiple rings and a liquid reservoir may be embedded into a contact lens platform 2600 as shown in Fig. 26. In an embodiment, a sensor 2614 with sensor material 2602, the sensor 2614 may be embedded in a contact lens platform 2610 distinguishes a liquid reservoir 2620 (amplifies the displaced liquid volume and show in this example as liquid reservoir rings), a gas reservoir 2630 and a sensing channel 2640 connected to the liquid reservoir 2620 on one end and to a gas reservoir 2630 on the other. First, liquid reservoir 2620 may be filled with a working liquid such as oil using capillary action and then sealed. This creates a stable gas/liquid interface 2650 in the sensing channel 2640 and forms a closed microfluidic network. The IOP fluctuations change the corneal radius of curvature; for every 1 mmHg increase in IOP, corneal radius of curvature increases 4 mm. This increases the liquid reservoir volume due to the strain applied on the liquid reservoir elastic walls. The increased reservoir volume creates a vacuum and shifts the gas/liquid position 2650 in the sensing channel 2640 towards the liquid reservoir 2620. As the sensing channel cross section area is reduced, the linear liquid displacement required to accommodate the reservoir volume change increases, hence the sensitivity improves. In some embodiments, the liquid may be water, saline or other pH balanced liquids suitable for use on or with the eye. In some embodiments, the liquid may be dyed a particular color to increase contracts with the air or increase or decrease contrast with the users eye color. In some embodiments, the contact lens platform may provide vision correction for the user. In some embodiments the contract lens platform may provide cosmetic color for the user. The color of the liquid in the microfluidic strain sensor may be dyed to enhance contrast or reduce contrast. In some embodiments, the air may be dyed to provide contrast instead, or in conjunction with the liquid.

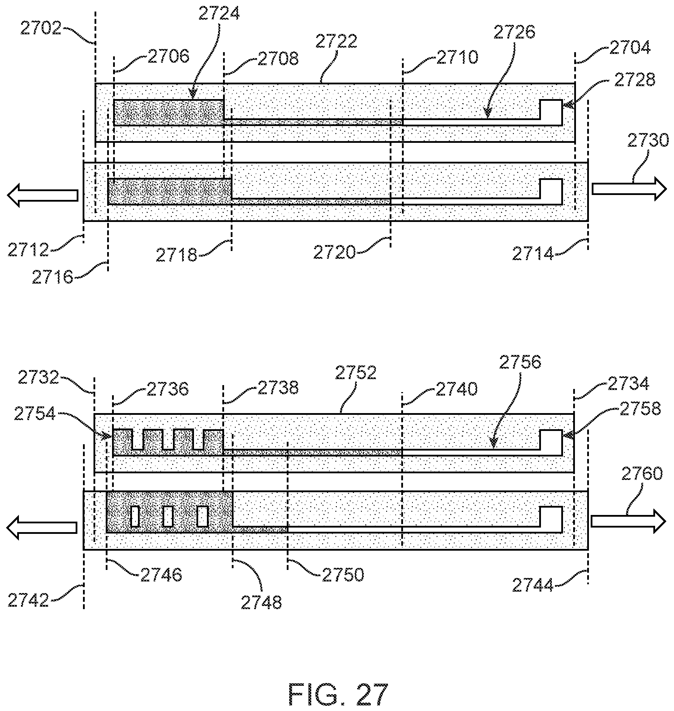

In various embodiments, the microfluidic strain sensor operates based on volume amplification of microfluidic liquid reservoir network when it may be stretched under tangential forces (Fig. 27). In some embodiments, the elements of the microfluidic strain sensor may be linearly distributed instead of radially distributed. In an embodiment, a side view of the microfluidic strain sensor with a liquid reservoir may be seen in Figure 27 and may have multiple chambers 2754 compared to a single wide chamber 2724. The sensor may be stretched under tangential forces. In an embodiment, a single liquid reservoir may have a width set between a first boundary 2706 and a second boundary 2708. When the sensor may be subject to strain, the first boundary 2716 may be pulled away from the center of the liquid reservoir 2724, while the second boundary 2718 may also be pulled away from the center of the liquid reservoir. In some embodiments, the first and second boundaries may be shifted in a generally uniform or consistent manner. In some embodiments, one boundary may be shifted more than the other. In various embodiments, the distance between the first and second boundary may be greater when the sensor may be under strain, than when the sensor may be at rest, or a reduced amount of strain. Similarly, in various embodiments where the liquid reservoir may have more than one channel, the first boundary 2736 and second boundary 2738 in an unstrained or normal condition, may have a first width, while the aggregate width of the multi-channel liquid reservoir may be wider as defined by the strained first boundary 2746 and second boundary 2748. Again, in various embodiments, the unstrained first boundary 2736 may have a similar position relative to the strained first boundary 2746. Likewise, the unstrained second boundary 2738 may have a similar position to the strained second boundary 2748. In the various embodiments, it may be the increase in the width between the boundaries (regardless of the relative position of any boundary to its strained or unstrained position) that may cause the liquid reservoir to take up more fluid under strain and cause the liquid-air boundary to shift from a first position 2710, 2740 to a second position 2720, 2750.

In the various embodiments, the strain sensor may have an air reservoir 2728, 2758 and an air filled portion of the sensing channel 2726, 2756. The various embodiments have a liquid filled portion of the sensing channel 2722, 2752 as well. In an embodiment, the strain sensor may have a circumferential direction of pull, as strain along the circumference of the eye may cause the contact lens platform to deform in all directions as the contact lens platform follows the contour of the eye itself. While the direction of pull 2730, 2760 may be observed in the illustration as being axial, the view is of a cross section of the generally circular sensor, and the actual direction of deformation may be in all directions of the contact lens platform as it sits on an eye.

In various embodiments, the strain sensor may have a first, unrestrained diameter, defined by the cross section boundaries 2702, 2704. When the strain sensor may be subject to strain, the cross section boundaries may expand slightly 2712, 2714. When the microfluidic sensor uses multiple liquid channels, the strain sensor boundaries may also change from a normal or unstrained set of boundaries 2732, 2734 to a strained set of boundaries 2742, 2744.

It should be remembered the figure presented is merely illustrative, to facilitate the understanding of the disclosure. In various embodiments, mechanical changes may occur when the closed microfluidic network is subject to tangential forces.

In some embodiments, the microfluidic strain sensor may experience a collapse. In an embodiment utilizing a single reservoir, the thin membrane above the liquid reservoir may collapse due to the induced stress and due to the low rigidity of the membrane. When multiple chambers with more rigidity membranes may be used, the collapse may not occur, or may decrease significantly. In various embodiments, the liquid reservoir volume may increase and produce a resulting vacuum effect. The liquid reservoir width may be elongated so the reservoir's volume may increase. If the membrane collapses, the volume increase may be reduced significantly. The volume increase may be amplified if the liquid reservoir consists of multiple chambers with small widths 2754. The amplification may be even higher if auxetic patterns exist on the membrane of the small reservoir chambers. When the volume of the liquid reservoir increases, the vacuum effect may pull the liquid/air interface position (3) towards the liquid reservoir. The movement of this interface, in µm, per IOP change, in mmHg, may be defined as sensitivity. Each 1 mmHg IOP change may cause a strain of 0.05%. This strain may cause approximately 100 µm position change on the interface position.

Another factor that may be considered for maximum sensitivity is the Young's modulus (E) of the sensor material. Increasing the E reduces the comfort of the wearer. When contact lenses with high lubricity may be used for improved comfort, the contact friction between the cornea and sensor/lens may decrease, which may cause slipping and decreased sensitivity, especially for high E sensors. Optimal E values may be obtained by additional experimentation. In some embodiments, the E value may be in the range of 0.2-10 MPa. In other embodiments, when the E value may be reduced below 2 MPa, the width of the reservoir channels may also be reduced to generally at or below 100 µm.

In some embodiments, the contact lens platform may be made with a non-fluidic strain sensor. According to some embodiments, the strain sensor may have a magnet, or ferro-magnetic material, embedded into or onto the contact lens platform. The magnetic material may respond to changes in a magnetic field, which may cause some depression or change in the cornea curvature. The change in the cornea curvature may be measured by determining the change in the magnetic material position relative to when the magnetic field may be off, or at a very low value. The change in the position may show the amount of magnetic force used to change the cornea (eye) curvature, and thus allow a determination of the resisting pressure (IOP) of the eye.

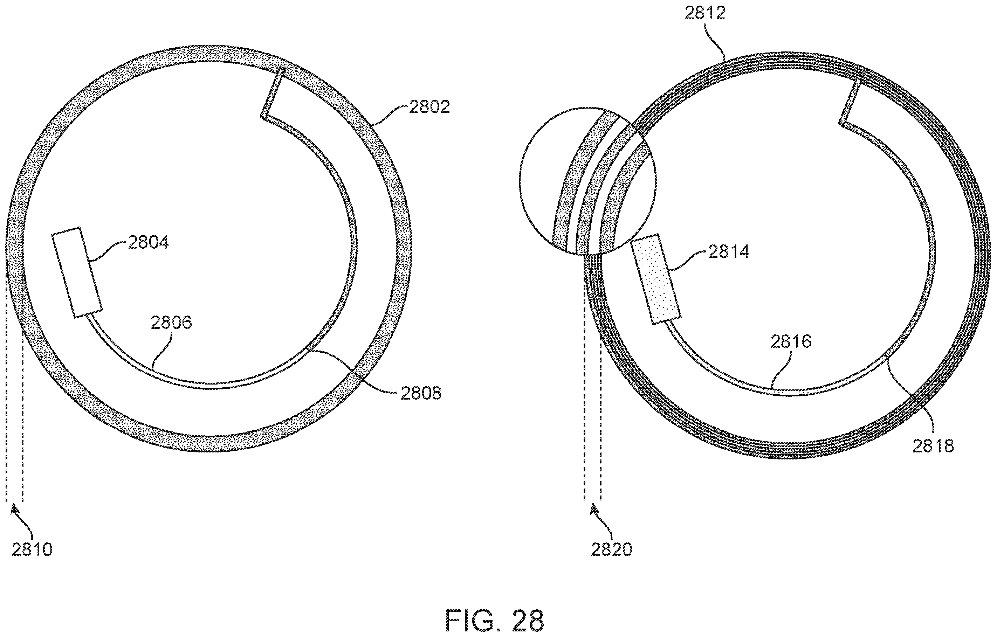

FIG. 28 shows the top view of two non-limiting embodiments. In an embodiment, a one ring 2810 reservoir with a single sensing channel are shown on the left. In another embodiment, a three ring 2820 liquid reservoir with a single sensing channel are shown on the right. Both of these, and other embodiments may be used as microfluidic strain sensors. In an embodiment, an increase of the vertical wall surface area of the liquid reservoir may increase the sensitivity of the sensor to changes in IOP. In various embodiments, increasing the number of walls and/or increasing the height of the channel walls may increase the sensitivity of the sensor. In other embodiments, the fluid reservoir may have a serpentine shape, oval or any other shape or pattern that may still provide the intended function as described herein. In some embodiments, the liquid in the fluid reservoir may have a tint, color or contract, such that the air-liquid interface may be more visible for image capture. In various embodiments, the liquid reservoir 2802, 2812 may contain a liquid, and the air reservoir 2804, 2814 may contain air. An air-liquid interface 2808, 2818 forms where the air and liquid meet. The position of the air-liquid interface may be used to determine the amount of strain the strain sensor may be experiencing.

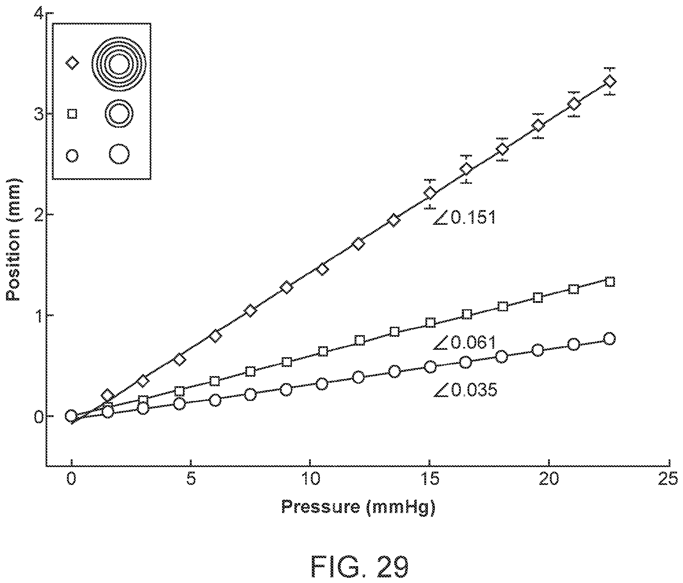

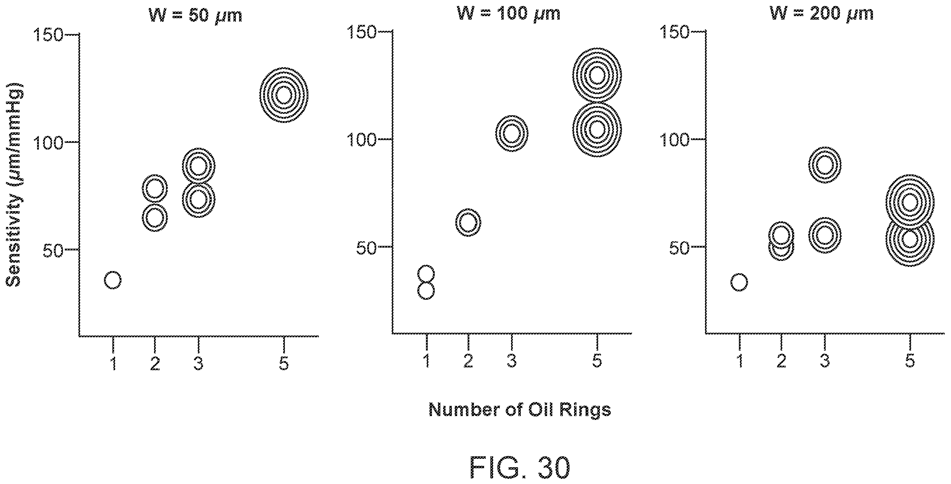

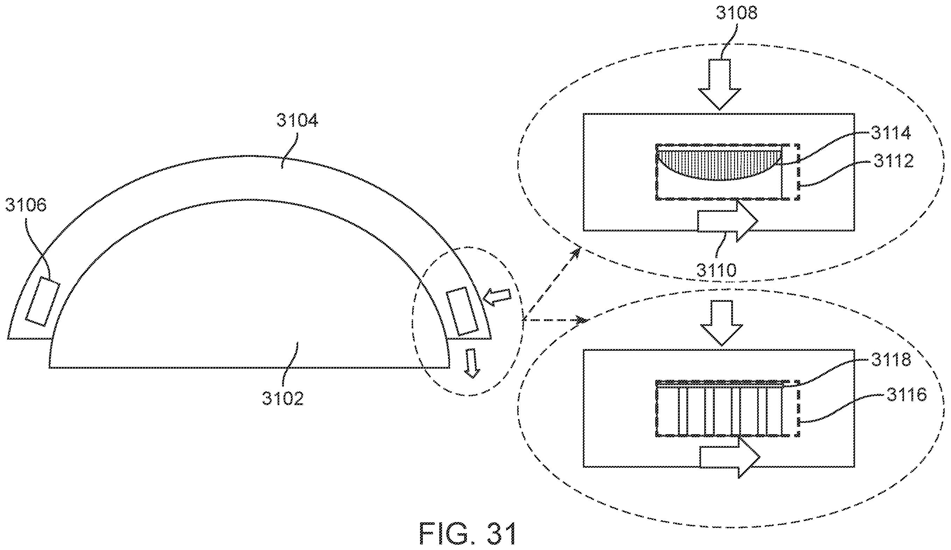

In various embodiments, the sensitivity results for a different number of rings are presented in FIGs. 29 and 30. The graph of Figure 29 illustrates the increase in the number of walls by adding more rings may also increase the sensitivity of the device in a linear manner. However, changing the width of the reservoir may not have a significant effect on the sensitivity of the sensor. This phenomenon may be a direct result of the interplay between tangential strain and radial force induced collapses as shown in FIG. 31. In various embodiments, an increase in the reservoir wall height may lead to an increase in sensitivity. In various embodiments, different heights were tested to determine differing sensitivities. Figures 29 and 30 are illustrations of some empirical test samples.

Figure 31 illustrates how different liquid reservoir patterns might behave under tangential strain (light arrow) and radial force (dark arrow). In an embodiment, the contact lens platform 3104 may have a liquid reservoir 3106 positioned outside the field of view of the eye. In an embodiment, the liquid reservoir 3106 may have one wide channel 3112. When the one wide channel may be subject to radial force 3108 and tangential strain 3110, the wide channel may deform by having the roof 3114 collapse into the channel and may decrease the sensitivity of the sensor. Alternatively, the liquid reservoir 3116 may be divided into smaller compartments and composed of multiple channels, with an optional patterning of the ceiling of one or more of the liquid reservoirs, then the collapses due to radial force (dark arrow) may be reduced.

In various embodiments, a variety of fabricated sensors with varying number of reservoir rings (1-5), ring widths (w = 50-500 µm), reservoir heights (50, 100, 330 µm) and chip thicknesses (130 µm, 300 µm) as well as different Young's moduli of about 1 MPa (PDMS) vs about 10 MPa (NOA 65) and about 100 MPa (NOA 61) were evaluated. The results of these sensitivity tests may indicate an increased liquid reservoir height increases the sensitivity of the sensor. In some embodiments, it may be possible to improve the sensitivity by adding more reservoir rings to the design as needed (e.g. depending on the required continuous wear contact lens properties). In still other embodiments, the stiffness (Young's modulus (E) x chip thickness (t) / width (w)) may not alter the sensitivity significantly; however, the stiffness may need to be optimized in view of other factors such as comfort and lens/cornea mechanic interactions.

Auxetic metamaterials for microfluidic strain sensing.

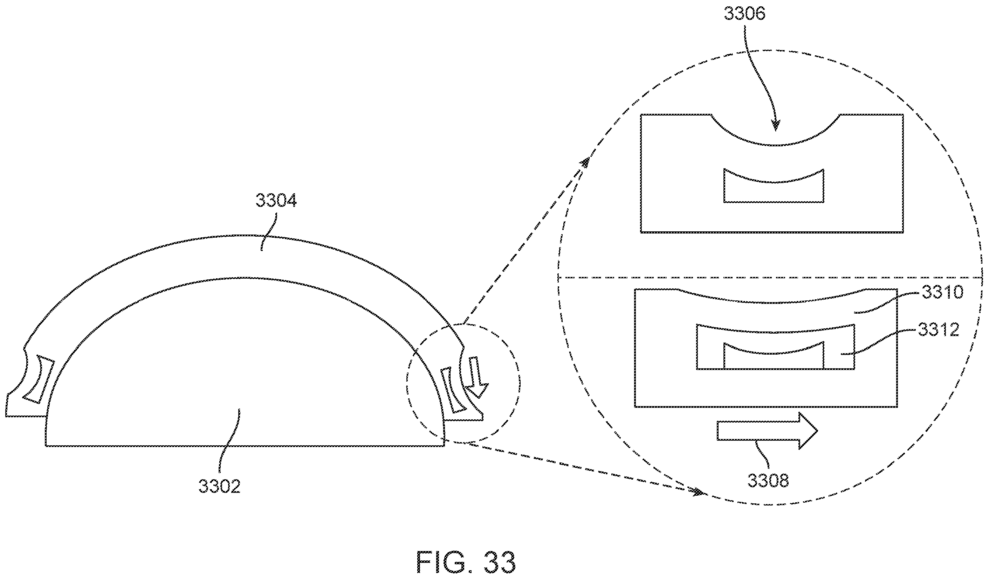

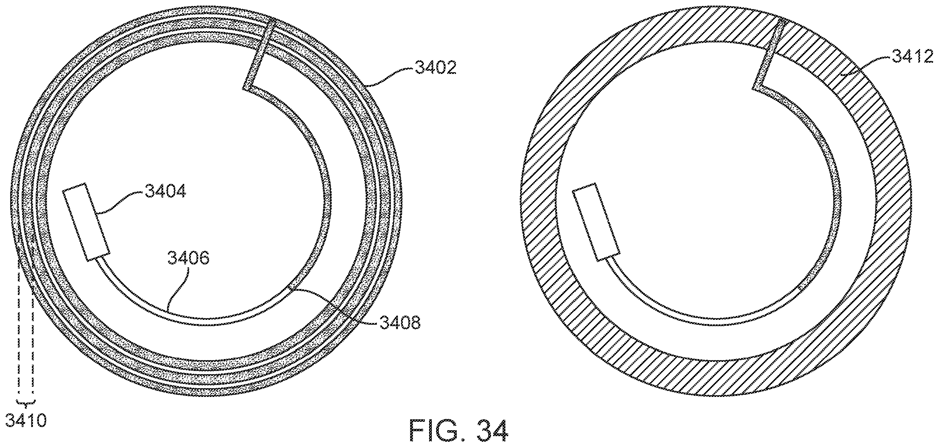

In an embodiment, the microfluidic channel network height may increase in response to the applied tangential strain 3310 (FIG. 33). The volume increase may be achieved by Poisson ratio modification through lithographical patterning of an elastomeric sensor. Figure 33 illustrates a cross-section of the contact lens sensor 3302 according to an embodiment. In this example embodiment, auxetic metamaterials may be used for strain sensing. The ceiling of the microfluidic channel may have a convex shape 3306, i.e., curved towards the channel interior, as shown. In some embodiments, this may be achieved by patterning the ceiling film 3412 with either circular or linear patterns as shown in FIG. 34. A tangential force may be applied (i.e., as when there are changes to the IOP of the eye) which may result in the ceiling deforming outward because of the convex ceiling, as opposed to the collapses observed when flat ceiling might be used. The deformation towards the front face of the sensor may cause a channel height increase, hence amplification in liquid reservoir volume expansion. This amplification may increase the sensitivity of the sensor.

In an embodiment, a sensor may have two or more circular fluid reservoirs 3402. The circular liquid reservoirs may be connected to form a single reservoir. The fluid reservoir rings may have a common or variable width 3410. An air reservoir 3404 may be connected to a microfluidic tube or channel, that has an air portion 3406 and a fluid portion. There may be an air-liquid interface 3408 demarking the position where the air and liquid meet in the channel.

In an embodiment, the fluid reservoir may have a physical relief pattern on one or more surfaces 3412 of the liquid reservoir. The patterning or relief features may help prevent the liquid reservoir from collapsing when subject to strain.

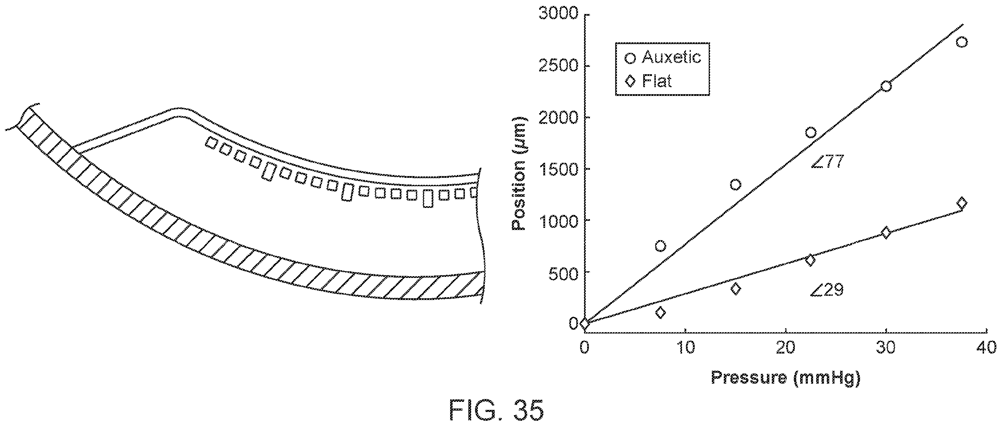

FIG. 35 on the left shows the image of the liquid reservoir on an auxetic sensor with a linear pattern of convex structures on the ceiling. FIG. 35 on the right shows the experimental sensitivity comparison between flat and curved (auxetic) devices according to various embodiment. An increase in sensitivity may be seen, up to a 2.5-fold increase.

In various embodiments, microfluidic mechanical metamaterials that may be biocompatible and electronics-free may enable fabrication of highly sensitive and reliable strain sensors. The tangential strain-sensing method disclosed herein may be specific to IOP as described herein. This approach was used to monitor IOP in porcine eyes and demonstrated generally a 1-mmHg detection limit (corresponds to 0.05% strain) and reliability over a test interval. The microfluidic strain sensor may measure the strain of the eye due to the shape changes in response to IOP in a clinically relevant range.

### Manufacturing.

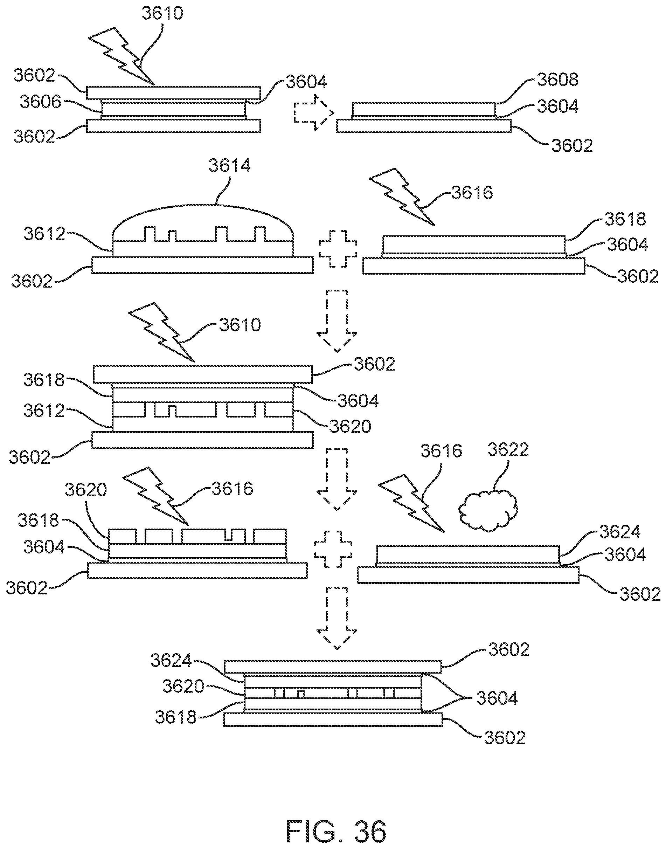

In some embodiments, the sensor may be made using photolithography and/or soft lithography techniques. In an embodiment, polydimethylsiloxane (PDMS) soft molds were fabricated and used to mold the sensor and contact lens platform. The sensor may be made from a polyurethane based Norland Optical Adhesive 65 (NOA65), which has favorable transparency, flexibility, oleophobicity and biocompatibility for various embodiments. Thin NOA65 films with the appropriate features may then be bonded together to make sensors as shown in FIG. 36. For the purposes of this disclosure, various equivalent fabrication methods made be used to create thin (~100 µm) microfluidic devices. The gas permeability of polyurethane used in the present disclosure may be up to 6-8 orders of magnitude lower than metals used in wearable electronics.

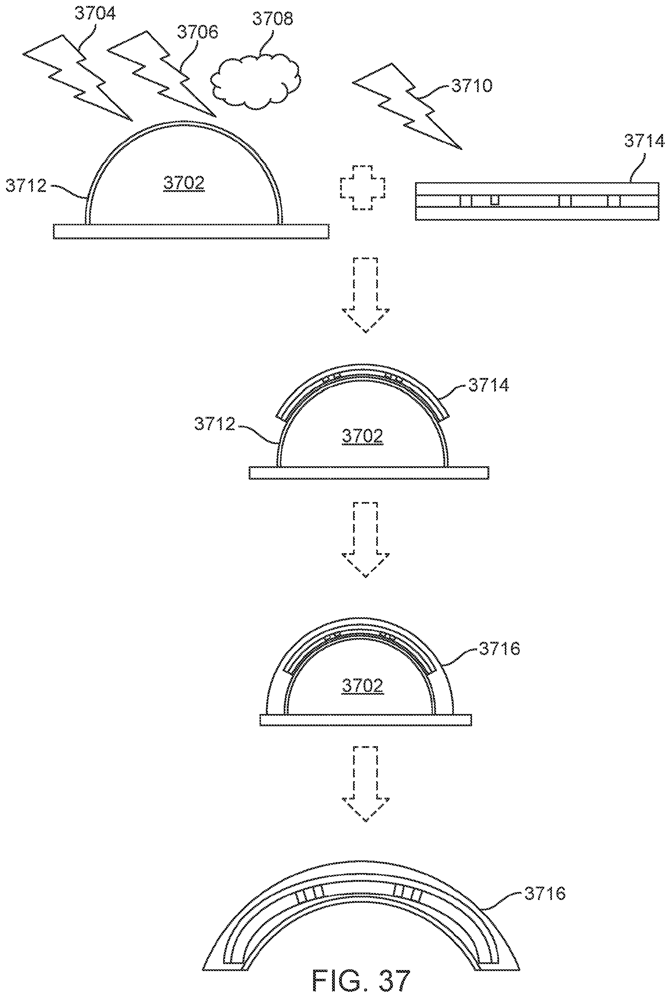

In an embodiment, the strain sensor may be cut into a particular shape and then embedded as a flat 80-120 µm strain sensor (FIG. 36) into a PDMS contact lens. In some embodiments, the sensors may be built curved if curved molds were used. The contact lens platform may be built with an 8-15 mm radius of curvature and a 10-14 mm radius as shown in FIG. 25. A dome shaped plastic mold may be used to pour PDMS on them to obtain a 10-150 µm silicone film at a particular radius of curvature. The sensor may be bonded on to the silicone film by (3-Aminopropyl) triethoxysilane (APTES) chemistry. More silicone may then be poured to fully embed the sensor in silicone. The details may be seen in FIG. 37. The contact lens platform may then be cut out with a circular puncher after curing the silicone at room temperature overnight. In various embodiments, the sensor may be made as thin as 50 µm thick so that overall contact lens sensor may be less than 150 µm.

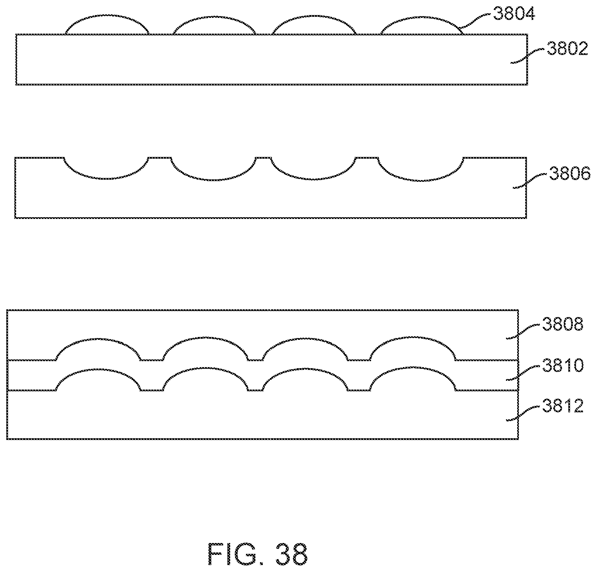

In another embodiment, an auxetic sensor version may follow the same manufacturing technique described above, with a variation in step 4 (Fig. 36), where a patterned film may be used instead of a flat film as the bottom layer. The patterning may be done as shown in FIG. 38. The master mold may be comprised of silicon wafer 3802 and positive resist 3804 may be used to fabricate negative 3806, 3808 and positive 3812 silicone molds. The negative 3808 and positive 3812 silicone mold pair may be used to fabricate patterned sensor layer 3810.

An example process of making the contact lens platform with an embedded microfluidic strain sensor is now shown in Figures 36 and 37. In an embodiment, an uncured UV curable adhesive 3606 may be sandwiched between two silicone layers 3604 on glass slides 3602. UV energy may be used to cure the adhesive 3606. A separate top silicone layer 3604 may be used to obtain a thin cured adhesive layer 3608. An uncured UV curable adhesive 3614 may be placed on a silicone mold with features 3612. A plasma treatment may then be applied to the surface of the blank cured adhesive layer 3618. A plasma treated blank cured adhesive layer 3618 and the mold 3612 with the uncured adhesive 3614 may be put together and a UV cure may be applied to the adhesive to bond the two layers together. The surface of the cured layer with the microfluidic layer may be prepared for bonding by using a plasma treatment, which may activate the surface. A plasma treatment 3616 of the surface of the blank cured adhesive layer 3624 may be used in combination with a APTES (3-Aminopropyl) triethoxysilane) treatment 3622.

The two activated layers may then be placed together like a sandwich for bonding.

Silicone may then be poured on a curved surface 3702 matching the size of a human cornea. The silicone may be cured by applying heat 3704. Additional plasma treatments 3706 and then APTES treatments 3708 may be applied to the surface of the silicone layer 3712. The treated surface of the sensor 3714 may then be placed on a curved silicone layer 3712 for bonding the two structures together. Another silicone layer may be applied on top and/or on the bottom of the silicone layer. The contact lens platform with an embedded microfluidic sensor may then be cut to size and finished.

It should be understood that other forms of strain sensors as described herein may be placed onto or into the contact lens platform using this or similar techniques, as will be readily apparent to those skilled in the art. Similarly, the sensor may be replaced with a thin or small magnet, or ferro magnetic material, which may be used with a magnetic field sensor.

### Variations and modifications.

In various embodiments, the microfluidic strain sensing technology of the present disclosure may be used for wide range of medical applications. Biomedical applications other than glaucoma management may include physiotherapy monitoring (e.g., at joints in hand injuries), speech recognition, fetus/baby monitoring, tremor diseases, robotics, and the like.

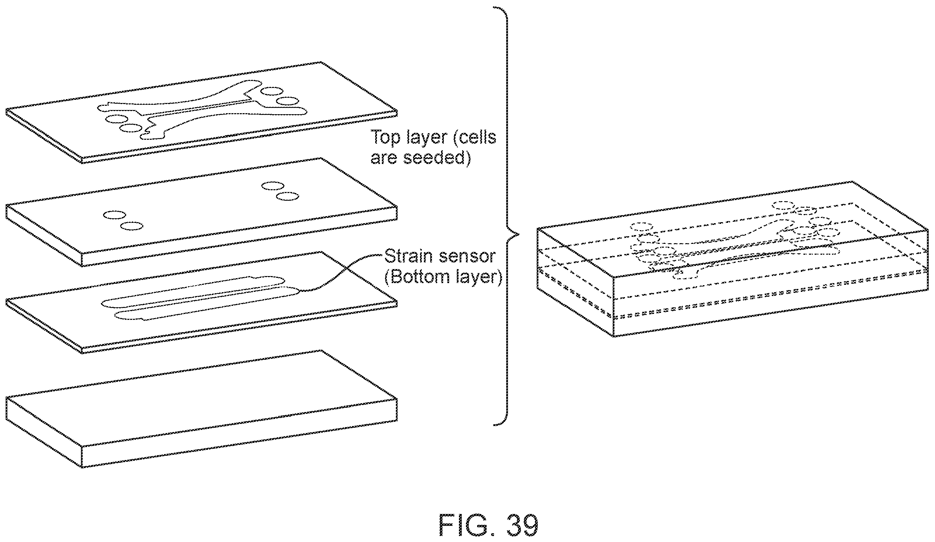

In various embodiments, microfluidic strain sensing may be used for biosensing and biochemical sensing as shown in FIG. 39. For example, it may be used to monitor or measure the strain applied by cells on a surface. Mechanical cues play important role in cellular processing such as cell differentiation, apoptosis, and motility. Cells senses and exert forces on a substrate where they grow. Tumor cells may generate more forces than regular cells. Shear stress, one of the leading physical cues may cause upregulation of genes activated by mechanic signals. Understanding mechanical cues generated by cells may help us to understand cancer progression. In some embodiments, a strain sensor as described herein, may provide direct monitoring of cancer cells signaling under exposure of different physical and mechanical cues. Therefore, it may bring a novel approach to cancer studies. In some embodiments, new biomarkers may be discovered, and/or new drug therapies could be implemented. The strain sensor as described herein may help in several other conditions including regulation of synaptic plasticity of neurons as forces are one of the key factors for progress of synaptic plasticity.

In an embodiment. two layers of microfluidic channels may be built as shown in FIG. 39. As cells grow, the strain sensor on the bottom channel may provide tissue stiffening. The top channel may also be manipulated by applying different flow rate which may change the shear stress. The cells mechanical response may be observed while they may be mechanically manipulated. This embodiment may be used in biomarker and drug development.

In another embodiment, a microfluidic strain sensor may be useful in studying cancer tissues as they progress and show more stiffer character. On average, cancer cells may be 4 times stiffer than regular cells. Understanding earlier stiffness of cancer cells may lead to earlier cancer detection. The strain sensor may be incorporated into patches which may be externally used on the skin. Specifically, the strain sensor may be used in skin and breast cancer types. Such patches with infrared beads embedded in microchannel may be optimized and implanted to internal organs in the case of ovarian cancer, liver and brain cancers. In some embodiments, these patches may be implanted after severe tumor removal surgeries to monitor cancer reoccurrence. Combining microfluidics-based strain sensors with flexible silicon electronics may enable multiplexed measurements on three dimensional soft tissues in vivo. This signal may be transferred to cloud-based system using wi-fi embedded technologies. Overall, the strain sensors incorporated with advance electronics may provide continuous monitoring of tissues which carries high chance of cancer reoccurrence.

In some embodiments, the microfluidic strain sensor may be manufactured by embedding the strain sensor with the desired shape/size in a contact lens. In some embodiments, the microfluidic strain sensor may be produced by directly patterning the desired topographies on the surface of the contact lens through soft lithography where features on a mold may transfer to a contact lens.

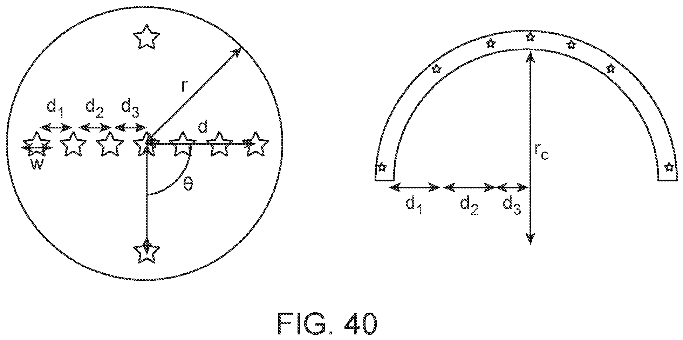

In an alternative embodiment, the distance between the microscopic geometric features on the contact lens may be directly measured instead of using microfluidics. This distance may change as a function of IOP. The geometric shapes and patterns of these features may be carefully selected to maximize the sensitivity to IOP. The IOP may be measured based on the imaging of contact lens sensor with geometrical features. FIG. 40 shows the top and side views of an example contact lens according to an aspect of the alternative embodiment. The location and shapes of the microscopic features for IOP determination are illustrated. The shapes in Fig. 40 are merely illustrative. Any shape or shapes, indicia, marker or fiducial may be used so long as a useful measurement may be taken from the sensor. In the top view, the radius of the contact lens is denoted by r and the value of r may be between 0.5 and 1 cm. Theta (θ), shows the angle between the features positioned at the periphery of the contact lens and it determines the number of features that may be placed angularly on a contact lens. The values for θ may be between 10 degrees (36 features at the periphery) and 180 degrees (two features at the periphery). In some embodiments, two or more features may be used on the contact lens. Symbols d₁, d₂, d₃,... dₙ may denote the distances between consecutive features and may be between 0.01 to 1 cm. The total distance d= d₁ + d₂ + d₃ +... +dₙ may be smaller than r. The radius of curvature of the contact lens, r_{c}, shown in the cross-sectional view may be between 0.5 to 1 cm. The characteristic width of features, w may be 0.001 to 0.5 cm.

As the IOP changes, the distances between peripheral features, e.g., d₁, may change and may be used as a measure of the IOP change. The distances between central features, e.g., d₂ or d₃, or the width of any feature, w, may be used as a reference measurement because they may not change in response to IOP. The distance between the opposing features at the periphery 2d changes the most as response to IOP change. The distance of any one of the contact lens features to the known features of the eye (i.e. iris border) may be detected as a measure of IOP.

Various aspects of the alternative embodiment have been tested and determined to function as described herein. In one example embodiment, a contact lens was made of PDMS and has thickness of about 250 µm. The contact lens was used on an eye model. The radius of curvature of the eye model changes of about 4 µm/mmHg (3 µm/mbar), mimicking the behavior of a human eye.

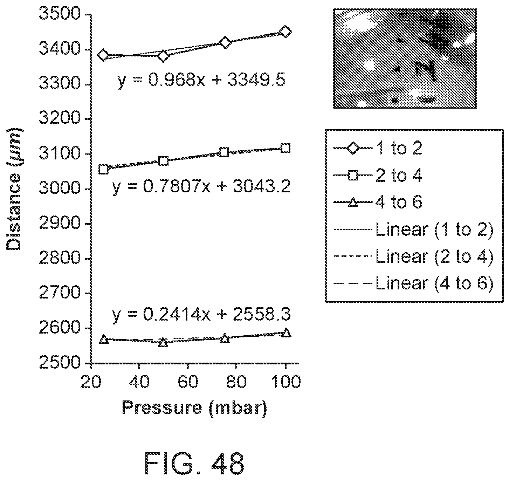

In some embodiments, marks may be placed on the contact lens. These marks may serve as probes and enable the measuring of the change in distance between different locations on the contact lens as a function of applied pressure as seen in Fig. 48. In one non-limiting example, four levels of applied pressure in the eye model used pressure varying from 25 mbar to 100 mbar. The contact lens was sampled at four locations, forming three distance measurements. The distances between these locations were plotted as a function of applied pressure as shown. The point located on the center of the contact lens was labeled as location '1' and the number was increased as the points located further from the center (e.g., location '2'). The distances between different marked points (e.g., location '1' to location '2') were measured. The triangle, square and diamond data plots show three lines showing the distance as a function of applied pressure for location 1 to 2, location 2 to 4, and location 4 to 6, respectively. Corresponding linear fits were plotted as well. Overall, the preliminary results show that the distances between different locations on the contact lens using distance markers follow a generally linear function of applied pressure, which falls into a measurable range.

In some embodiments, the contact lens platforms with distance markers may be fabricated similar to strain sensor contact lens or they may just be marked with an ink.

In some embodiments, the contact lens device may be used as a temperature sensor as since thermal expansion of any material may produce strain on the strain sensor, or cause deformation of the distance sensor, allowing a determination of the thermal change of an object by analyzing the change on the sensor. In some embodiments, the sensor may not be a contact lens, but may be shaped to conform to the surface of the object to be measured, whether for a thermal measurement, strain sensing, growth sensing of a cellular mass, or any other function. In some embodiments, the strain sensor/distance sensor may be used in vacuum, e.g., in space applications, as the sensor may not be negatively affected by low or zero air pressure.

In some embodiments, the images may be taken by a smartphone camera, a special handheld camera, or by a wearable camera. The images may be taken directly across the eye, at any angle between 0 to 180 degrees. In some embodiments, the images may be taken automatically by a smart phone when a patient may be using their smart phone, so the image capture may be done passively (without active participation by the patient).

### Additional Technical Notes

In some embodiments, the closed microfluidic network for strain sensing may have a strain sensitivity of 2-15 mm interface movement per 1% strain depending on the number of rings used in the strain sensor. The sensitivity may be increased by increasing the number of rings. The strain sensor may be made robust enough to withstand pressure changes that are applied for 24 hours. In addition, the strain sensor may have a lifetime of several months under normal usage. In various embodiments, extreme strain levels smaller than 0.1% may be measured by allowing the sensor to sit on the surface to be measured, for an extended period of time. That period of time may be a few minutes to a few hours to a few days. In some embodiments, the embedded sensor of a contact lens platform allows for the strain sensor to sit on the surface of the eye for an extended period of time, allowing for the monitoring of intraocular pressure (IOP). In some embodiments, IOP sensing may be 1 mmHg. This value may correspond to a strain of 0.05%. The microfluidic strain sensor may achieve this strain detection requirement by designing a liquid reservoir network that includes multiple microfluidic channels as a liquid reservoir. The liquid reservoir network may be connected to a sensing channel and the sensing channel may be connected to an air reservoir. In various embodiments, these three components may form a closed system. In an embodiment, the microfluidic sensor may be filled from the inlet with a working liquid, using only capillary forces. When the working liquid reaches the outlet, both inlet and outlet may be sealed using the sensor material to form a closed system with a fixed liquid volume inside. The liquid may fill the sensing channel to approximately half of its total length, creating a liquid/air interface. Both the contact lens and the sensor may be made of elastomers such as silicone and polyurethane but may be made of other materials such as silicone/hydrogel.

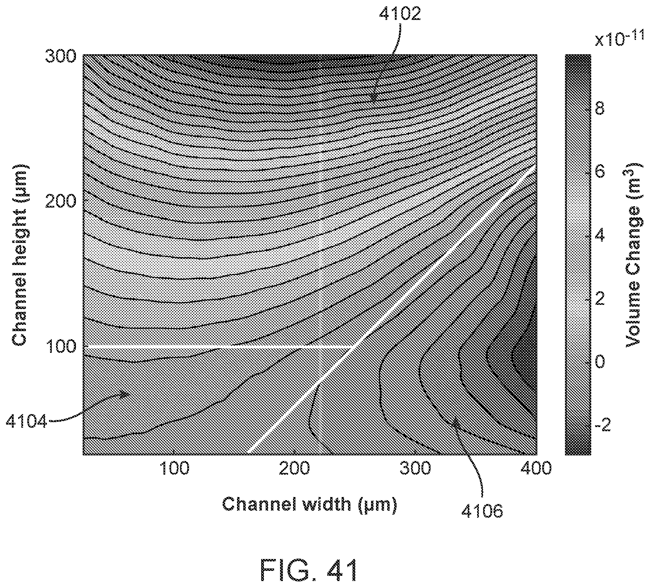

A heat map for the volume increase of a microfluidic strain sensor with a membrane thickness of about 20 µm for different channel height and width values is shown in Fig. 41 according to an embodiment. The map illustrates three different operation zones representing: i) collapse 4106, ii) thin 4104 and iii) thick sensors 4102. According to an embodiment, the collapse zone may correspond to a malfunctioning device due to reduced sensitivity and unstable interface movement. Even though zone iii) 4102 may have a high-volume change and stable operation, it may not be practical for many applications. The large thickness of the sensor may reduce both the comfort and functionality. Generally thin devices may be better suited for biomedical applications, and in particular for use on the cornea. In some embodiments, a thin sensor, corresponding to zone ii) 4104, may demonstrate high volume change when subjected to low amounts of strain, and thus may be selected for the fabrication of strain sensors as described herein. In various embodiments, the channel height and membrane thickness may be dictated by the sensor thickness, while the channel width may be the main parameter to control for maximizing the volume change. In some embodiments, a width of about 50 µm may produce good results.

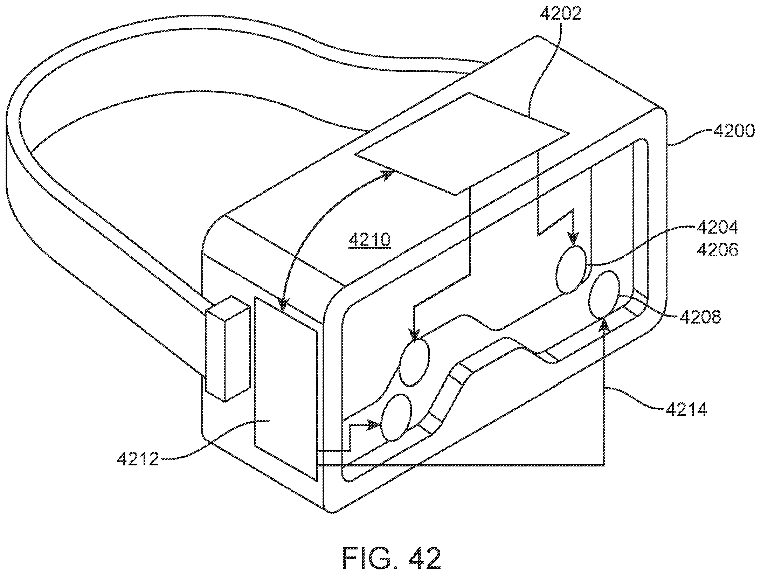

In some embodiments, there may be an eye wear device 4200, which may be a pair of glasses, goggles, or other gear designed to be worn over or in close proximity to the eyes as shown inf Fig. 42. The eyewear device may have a snug fitting around the eyes (like ski goggles) or a more open environment for the eyes like a pair of glasses. In some embodiments, the eye wear device may cover or be in close proximity to, one eye, or both eyes. In some embodiments the eye wear device may be removable device that may fit over or in close proximity to, a pair of glasses or other eye wear. The eye wear device 4200 may be referred to herein as a pair of goggles (or simply goggles) and the term should be understood to mean an eye wear device. The goggle may have an imaging system and/or a drug delivery system. The goggle may also have an onboard electronic controller, such as a computer chip, micro-chip or any other electronic device for monitoring the imaging system, determining when to dispense a medication from the drug delivery system, and causing the drug delivery system to deploy medication. In some embodiments, the image capture system may be any image capture system described herein. In some embodiments, the image capture system may use two or more imaging devices or systems as described herein. In still other embodiments, the imaging system may be any equivalent imaging device, system, electronic, or mechanism that may work with the goggles and the drug delivery systems disclosed herein.

In some embodiments, the goggles 4200 may have an image acquisition controller 4202. The image acquisition controller may also provide wireless connectivity with an external electronic device, such as a mobile phone, tablet, computer or cloud-based device or system. In some embodiments, the wireless connection may be an antenna or a wireless circuit, with control over frequency and bandwidth transmission, so as to transmit over short-range wireless systems like BlueTooth or WiFi. In some embodiments, the wireless connectivity may be a cellular connection, or similar long range communication protocol. The image acquisition controller may be in electronic communication with a camera 4204 or a light source 4206. The camera 4204 may be a CCD or high-definition image sensor. In some embodiments, the image sensor may be an electromagnetic sensor. The light source may be a single point light source, or a set of lights of similar or different types. The light source may be an array, or a series of light sources of common or different wave lengths. The light source may be activated by the image acquisition controller, or a separate light source controller. The activation of the light source may be all at once, in parallel (groups of lights going off at once), in series (one light going off after another), or any sequence of turning light sources on or off that may be programmed into the light source controller or image acquisition controller. In some embodiments, where the sensor may rely on other forms of electromagnetic energy besides visible light, a light source may not be part of the goggles or eyewear device 4200. In some embodiments, the goggles may be able to detect a variety of different strain sensors. The goggles may have the appropriate sensor for each type of strain sensor employed by the patient. In some embodiments, the contact lens platform may be used to induce a shape change on the eye, and the sensor of the goggle may be used to detect the induced shape change, and the different shapes of resting versus induced change, may be used to determine the IOP of the eye.

In some embodiments, the goggles 4200 may also have a medication dispenser or a drug delivery system 4208. In some embodiments, the drug delivery system may be a device that atomizes or nebulizes a fluid mixture of a medication. In some embodiments the drug delivery device may be a piezoelectric driven nebulizer. In some embodiments there may be a second drug delivery device 4210, which may be the same type of device as the first drug delivery device 4208. In some embodiments, the second drug delivery device 4210 may be a different type of device, dispense a different medication, operate independently of the first drug delivery device, or work in combination with the first drug delivery device. In another embodiment, the goggles 4200 may have one or more drug reservoirs 4212. In yet another embodiment, the goggles 4200 may have wiring and/or fluid tubing, to provide electrical communication between any components using electrical energy, and to connect elements that are in fluid communication. The presence of the tubing 4214 is merely illustrative, and the wiring, tubing or circuitry for the goggles may be laid out in any functional or cosmetic fashion.

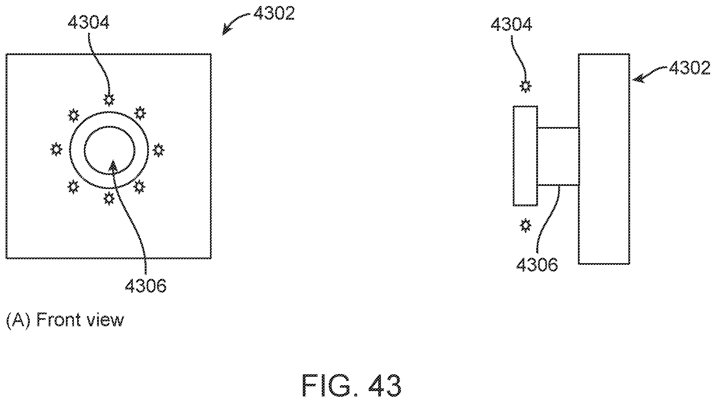

In an embodiment, the image capture device 4302 and the light source 4304 may be unified into a single system, as shown in Fig. 43. In an embodiment, the image capture device may be a camera and illumination system may be a circular constellation of LEDs placed around a camera lens. The LEDs may provide the lighting needed to observe an IOP measuring device, such as a contact lens on the cornea of an eye. In an embodiment, the camera may be connected to the controller. In some embodiments, the images acquired by the camera may be transmitted through a wireless or wired link to a remote imaging processing device, such as a mobile phone, a tablet, a desktop computer, a mobile computer or a cloud computer.

In some embodiments, the eye wear device may include an image capture device 4302. The image capture device may be a light sensitive device, or an actual imaging device, like a camera. The image capture device 4302 may have a light source 4304. In some embodiments, the light source may be a single point source of light. In some embodiments, the light source may be multiple light emitting elements, such as LEDs. In some embodiments, the light source may be an array of LEDs, which may be arranged as an annular array, a square array or a linear array. In still some other embodiments, the LED array may be arranged in any sequence or shape. In some embodiments, the light source may emit light in a single wavelength, or a narrow band of wave lengths. In other embodiments, the light source may emit light in a wide spectrum of light (wide frequency) with each light of the light source producing a broad spectrum of light, or each light in an array emitting a light of a different frequency. In some embodiments, the imaging sensor may be a camera, and the camera may have a lens 4306. In some embodiments the lens may be treated with a coating to reduce fogging up of the lens. In some embodiments the lens may be coated with an anti-glare material, so as not to reflect light to a user wearing the goggles.

In an embodiment, the camera and illumination system may be a circular constellation of LEDs 4304 placed around a camera lens 4306 as shown inn Fig. 43. The LEDs may provide the lighting needed to observe an IOP measuring device, such as a contact lens on the cornea of an eye. In an embodiment, the camera may be connected to the controller 4302. In some embodiments, the images acquired by the camera may be transmitted through a wireless or wired link to a remote imaging processing device, such as a mobile phone, a tablet, a desktop computer, a mobile computer or a cloud computer.

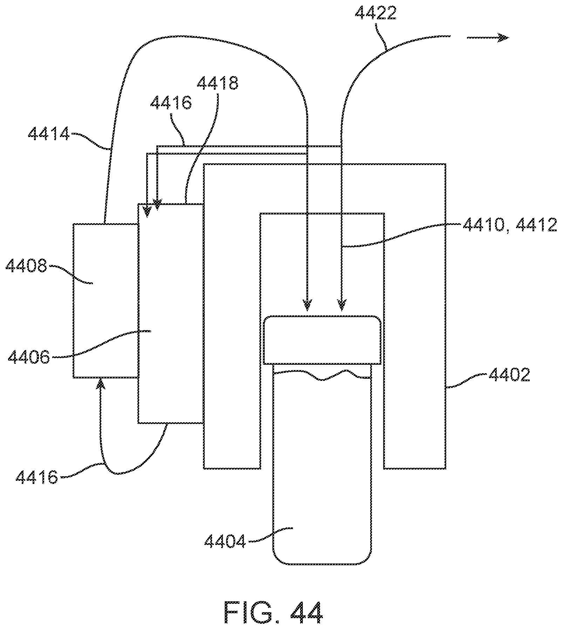

In an embodiment, there may be a drug delivery system for use with the goggles 4200 as shown in Figures 44-45. In an embodiment, the drug delivery system may have a medication storage component 4400 and a medication mist generator component 4500.

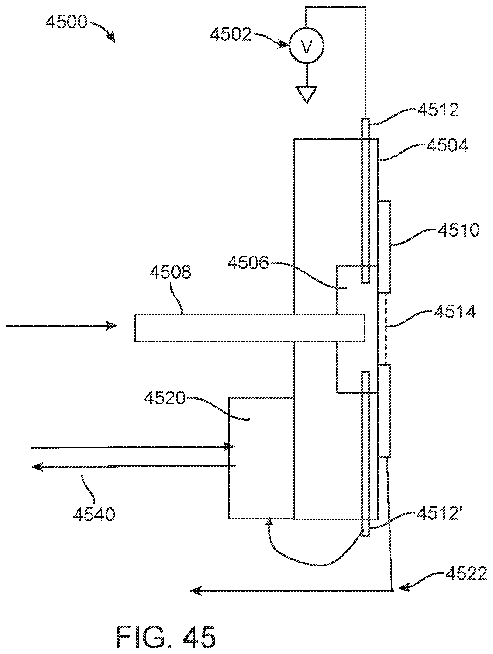

In an embodiment, the drug delivery system may have a storage component 4400 for storing a medication or drug for the treatment of a person's eye. In an embodiment, there may be a body 4402 having a fastener or aperture for receiving or holding a drug reservoir 4404. In some embodiments a control circuit 4406 may be attached or incorporated into the body 4402. In some embodiments, the control circuit may be part of the controller used for the goggles. In an embodiment, a pressure generating pump 4408 may be attached to the body. A first needle may be integrated into the body 4402 so that when a drug reservoir 4404 is attached to the body, the first needle 4410 may puncture the drug reservoir. The first needle 4410 may be connected to the pressure generating pump 4408 such that if the pump is activated, air or other material may be pumped into the drug reservoir 4404 and generate a positive pressure environment inside the drug reservoir. A second needle 4412 may be used to penetrate into the drug reservoir, and allow the drug to flow into a second fluid conduit that channels the drug solution to a mist generator. In an embodiment, air may be used to pressurize the reservoir. Air may be pumped in using the pressure generating pump 4408 and enter the reservoir 4404 through a first needle 4410, then the drug or medication may be forced out through the second needle 4412. The pump 4408 may be connected to the first needle 4410 with a hose 4414 or other tubing, allowing positive pressure to be created in the reservoir. A septum or other device may be used to prevent the loss of pressure in the reservoir. The first and second needles may also have flow control elements to prevent the loss of reservoir pressure. In some embodiments, there may be electrical wiring, electrical circuitry or other conductive elements 4416 to permit the flow of electrical signals and electrical power to and from the various electrical components and a power source. The wiring may be integrated into the goggles, or the wiring 4416 may be free standing and removable or adjustable separate from the goggles. In some embodiments, a power source (not shown) may be connected to the pump 4408 and the control circuit 4406. In some embodiments, there may also be a tube or hose to convey the medication from the second needle 4412, through a second hose 4422, to a nebulizer device 4500 (Fig. 45). In some embodiments, there may be an electrical connection between the control circuit 4406 and the nebulizer device 4500. In other embodiments, there may be an electrical connection between the goggle controller 4202 and the nebulizer device 4500. In some embodiments, there may be electrical communication between both a control circuit 4406, a main controller 4202, and the nebulizer device 4500.

In an embodiment, the control circuit 4406 may monitor the level of a drug present in the reservoir 4404 through the needles inserted into the reservoir. In some embodiments, the needle(s) may act as sensors, using a capacitive and/or electrochemical signal to provide data to the control circuit 4406, or main controller 4202, so either control device may determine the level of the drug in the reservoir 4404.

In an embodiment, a nebulizer device 4500 may be in fluid and/or electrical connection with a medication storage component 4404. In some embodiments, the nebulizer device may have a body 4504. The body 4504 may have a fluid cavity 4506 which may hold a drug or medication delivered to the fluid cavity 4506 through a drug delivery tube 4508. In some embodiments, the drug delivery tube 4508 may be connected to a drug reservoir. In some embodiments, the fluid cavity 4506 may have an opening, or a port. The port may be partially covered by a mist generator 4510. The mist generator may have a perforated section 4514 where the perforation holes or apertures may be sufficiently small to prevent fluid from moving through the holes without assistance. In some embodiments, the mist generator may vibrate, causing the perforated section to vibrate, and produce a mist of the fluid in the fluid cavity. In some embodiments, a sensor 4512 may measure the volume of the fluid in the fluid cavity 4506. There may be a single sensor 4512, or any number of additional sensors 4512'.

In some embodiments, the mist generator may be a piezoelectric element, like a transducer, that may vibrate at a particular frequency and intensity. The vibration of the mist generator 4510 may cause the perforated section 4514 to vibrate as well. The amplitude and frequency of the vibration may produce an interaction with the medication or drug in the fluid cavity 4506. The interaction may cause the fluid to eject through the perforation in the perforated section 4514 and produce droplets. The size and frequency of droplet production may be varied by the size of the apertures in the perforated section, along with the amplitude and frequency of the vibration used in the mist generator 4510. In some embodiments, the amplitude and frequency may be programmed into any one or more controllers that may control the mist generator. In some embodiments, a preamplifier 4520 may be used to drive the mist generator 4510. The mist generator 4510 may be electronically connected 4522 to a main controller or a secondary controller or connect to both. The various components using electrical energy, receiving or sending electronic signals may be connected electronically.

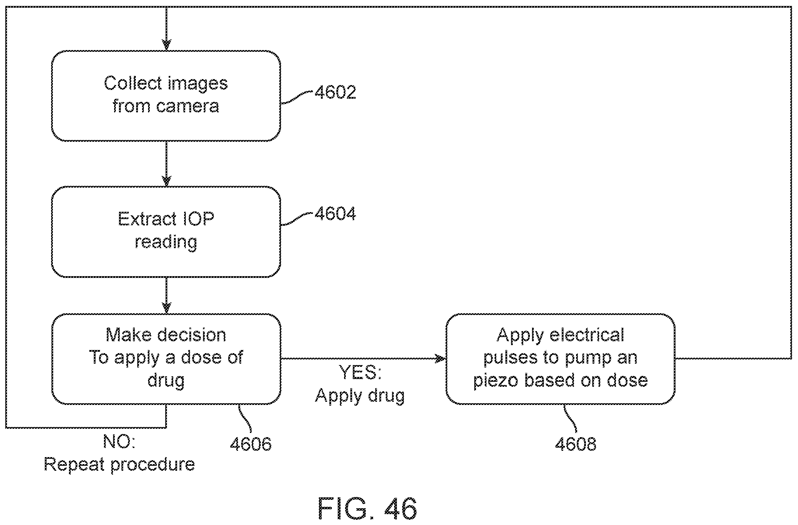

In an embodiment, the drug dispensing device may follow a flow chart for decision making as shown in Fig. 46. In an embodiment, a sensor may monitor the contact lens as described herein. In some embodiments the sensor may be an optical sensor. In some embodiments, the sensor may be a magnetic field sensor. In an embodiment, the optical sensor may provide the optical image data to a processor 4602. The processor may convert the optical image data into an IOP reading, or IOP data 4604. Alternatively, if the sensor may be a magnetic field sensor, the sensor may detect the position of a magnet or magnetic material within a magnetic field. The position of the magnet in the magnetic field may correspond to an indentation of the eye surface, which may also be used to calculate the intraocular pressure of the eye. When the IOP data meets a predetermined threshold 4606, the processor may activate the drug dispensing system 4608 of the goggle. The drug dispensing system may have a series of different doses to provide, based on different signals from the processor. The drug dispensing system may deploy the proper medication, in the proper volume, to the volume of space in close proximity to the eye. The presence of the aerosolized medication near the eye may then be absorbed by the eye. The medication may be sprayed toward the eye, presented as a mist near the eye for gradual absorption by the eye, or streamed into the eye. Droplet and stream size may be controlled by modifying the frequency of the ultrasound transducer, and the size of the pores on the surface of the fluid cavity.

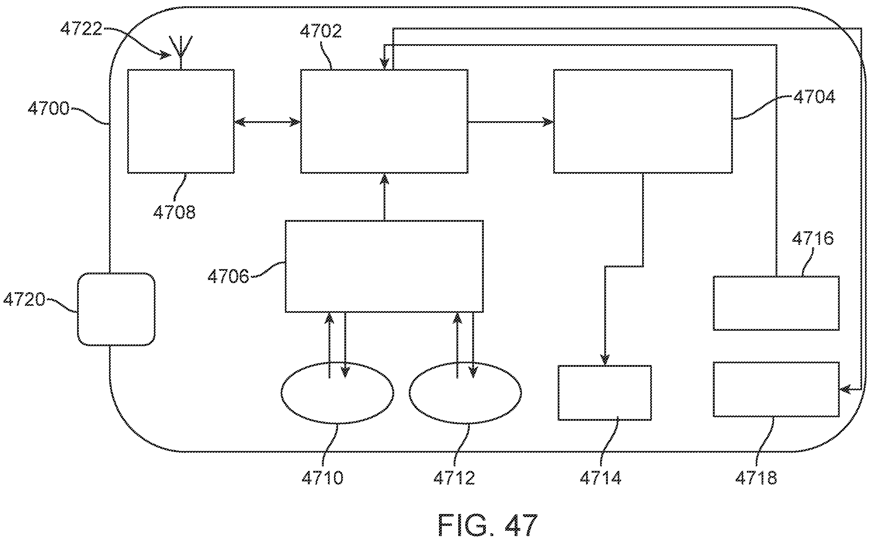

A schematic of a wearable eye gear with a drug delivery system and IOP sensor is now shown in Fig. 47. In an embodiment, the eye wear may have an electronic circuit 4700 with a controller 4702, a wireless connectivity module 4708 (which may include an antenna 4722), a nebulizer with drive electronics 4704, and a fluid sensor 4706. In an embodiment, the eye wear gear may have a drug reservoir 4710, and a nebulizer cavity 4712. A piezoelectric element 4714 may be positioned on, in or around the nebulizer cavity 4712 to generate a mist of the drug or medication provided from the drug reservoir. In some embodiments, the eye wear gear may have a sensor 4716, and a light source 4718, which may be LEDs, a laser or some other light generating device. A power cell 4720 may be connected to the circuit 4700 to provide power to the electrical components. In some embodiments, the sensor 4716 may be an image sensor, and light source 4718 may be activated so a contact lens with a strain gauge may be illuminated and read. In some embodiments the sensor may be a magnetic field sensor, and the magnetic field sensor may be activated to detect the position of a magnet or magnetic material in or on a contact lens platform. The sensor may capture the image of the strain gauge, or determine the position of the magnet in the magnetic field, and send the data to the controller 4702. The controller may evaluate the data and determine the IOP of the eye. The controller may then prime the nebulizer fluid cavity with a medication or drug from the drug reservoir 4710. Once the fluid cavity 4712 may be primed, the piezoelectric element 4714 may be activated, causing the medication/drug to be delivered to a volume in close proximity to the eye, or onto the eye/contact lens platform.

In an embodiment, the drug delivery system may be controlled by the onboard processor. In some embodiments, the drug delivery system may be controlled by a remote processor. In the various embodiments, the controller may be programmed to automatically dispense a drug when a certain IOP threshold may be detected, or dispense a drug on demand. The timing and dose value of the drug may be determined using an algorithm, a schedule or a combination of an algorithm and a schedule. The IOP readings may be reported to the cloud, which may be accessed by a doctor. The doctor may set a threshold value for delivering a dose, or a schedule for the delivery of a dose of the drug. The doctor may make a decision based on the history of the IOP readings to determine the threshold value above which a certain dose of drug might be applied. The drug application dose history may also play a role in the doctor's decision. When the IOP meets a certain threshold value, a dose may be applied automatically, by the patient, or by the doctor. This process may form the basis of an algorithm that allows full- automatic decision making for the threshold IOP value and dose value.

Embodiments of the subject matter and the operations described in this specification may be implemented in digital electronic circuitry, or in computer software, firmware, or hardware, including the structures disclosed in this specification and their structural equivalents, or in combinations of one or more of them. Embodiments of the subject matter described in this specification may be implemented as one or more computer programs, i.e., one or more modules of computer program instructions, encoded on one or more computer storage medium for execution by, or to control the operation of, data processing apparatus, such as a processing circuit. A controller or processing circuit such as CPU may comprise any digital and/or analog circuit components configured to perform the functions described herein, such as a microprocessor, microcontroller, application-specific integrated circuit, programmable logic, etc. Alternatively or in addition, the program instructions may be encoded on an artificially generated propagated signal, e.g., a machine-generated electrical, optical, or electromagnetic signal, that is generated to encode information for transmission to suitable receiver apparatus for execution by a data processing apparatus.

A computer storage medium may be, or be included in, a computer-readable storage device, a computer-readable storage substrate, a random or serial access memory array or device, or a combination of one or more of them. Moreover, while a computer storage medium is not a propagated signal, a computer storage medium may be a source or destination of computer program instructions encoded in an artificially generated propagated signal. The computer storage medium may also be, or be included in, one or more separate components or media (e.g., multiple CDs, disks, or other storage devices). Accordingly, the computer storage medium is both tangible and non-transitory.

The operations described in this specification may be implemented as operations performed by a data processing apparatus on data stored on one or more computer-readable storage devices or received from other sources. The term "data processing apparatus" or "computing device" encompasses all kinds of apparatus, devices, and machines for processing data, including by way of example a programmable processor, a computer, a system on a chip, or multiple ones, or combinations, of the foregoing The apparatus may include special purpose logic circuitry, e.g., an FPGA (field programmable gate array) or an ASIC (application specific integrated circuit). The apparatus may also include, in addition to hardware, code that creates an execution environment for the computer program in question, e.g., code that constitutes processor firmware, a protocol stack, a database management system, an operating system, a cross-platform runtime environment, a virtual machine, or a combination of one or more of them. The apparatus and execution environment may realize various different computing model infrastructures, such as web services, distributed computing and grid computing infrastructures.

A computer program (also known as a program, software, software application, script, or code) may be written in any form of programming language, including compiled or interpreted languages, declarative or procedural languages, and it may be deployed in any form, including as a standalone program or as a module, component, subroutine, object, or other unit suitable for use in a computing environment. A computer program may, but need not, correspond to a file in a file system. A program may be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program may be deployed to be executed on one computer or on multiple computers that are located at one site or distributed across multiple sites and interconnected by a communication network.

The processes and logic flows described in this specification may be performed by one or more programmable processors executing one or more computer programs to perform actions by operating on input data and generating output. The processes and logic flows may also be performed by, and apparatus may also be implemented as, special purpose logic circuitry, e.g., an FPGA (field programmable gate array) or an ASIC (application specific integrated circuit).

Processors suitable for the execution of a computer program include, by way of example, both general and special purpose microprocessors, and any one or more processors of any kind of digital computer. Generally, a processor will receive instructions and data from a read only memory or a random access memory or both. The essential elements of a computer are a processor for performing actions in accordance with instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer may be embedded in another device, e.g., a mobile telephone, a personal digital assistant (PDA), a mobile audio or video player, a game console, a Global Positioning System (GPS) receiver, or a portable storage device (e.g., a universal serial bus (USB) flash drive), to name just a few. Devices suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto optical disks; and CD ROM and DVD-ROM disks. The processor and the memory may be supplemented by, or incorporated in, special purpose logic circuitry.

To provide for interaction with a user, embodiments of the subject matter described in this specification may be implemented on a computer having a display device, e.g., a CRT (cathode ray tube) or LCD (liquid crystal display) monitor, OLED (organic light emitting diode) monitor or other form of display for displaying information to the user and a keyboard and/or a pointing device, e.g., a mouse or a trackball, by which the user may provide input to the computer. Other kinds of devices may be used to provide for interaction with a user as well; for example, feedback provided to the user may be any form of sensory feedback, e.g., visual feedback, auditory feedback, or tactile feedback; and input from the user may be received in any form, including acoustic, speech, or tactile input. In addition, a computer may interact with a user by sending documents to and receiving documents from a device that is used by the user; for example, by sending web pages to a web browser on a user's client device in response to requests received from the web browser.

While this specification contains many specific embodiment details, these should not be construed as limitations on the scope of any embodiments or of what may be claimed, but rather as descriptions of features specific to particular embodiments. Certain features described in this specification in the context of separate embodiments may also be implemented in combination in a single embodiment. Conversely, various features described in the context of a single embodiment may also be implemented in multiple embodiments separately or in any suitable subcombination. Moreover, although features may be described above as acting in certain combinations and even initially claimed as such, one or more features from a claimed combination may in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems may generally be integrated in a single software product or packaged into multiple software products.

References to "or" may be construed as inclusive so that any terms described using "or" may indicate any of a single, more than one, and all of the described terms.

Thus, particular embodiments of the subject matter have been described. Other embodiments are within the scope of the following claims. In some cases, the actions recited in the claims may be performed in a different order and still achieve desirable results. In addition, the processes depicted in the accompanying figures do not necessarily require the particular order shown, or sequential order, to achieve desirable results. In certain embodiments, multitasking and parallel processing may be advantageous.

Having described certain embodiments of the methods and systems, it will now become apparent to one of skill in the art that other embodiments incorporating the concepts may be used. It should be understood that the systems described above may provide multiple ones of any or each of those components and these components may be provided on either a standalone machine or, in some embodiments, on multiple machines in a distributed system. The systems and methods described above may be implemented as a method, apparatus or article of manufacture using programming and/or engineering techniques to produce software, firmware, hardware, or any combination thereof. In addition, the systems and methods described above may be provided as one or more computer-readable programs embodied on or in one or more articles of manufacture. The term "article of manufacture" as used herein is intended to encompass code or logic accessible from and embedded in one or more computer-readable devices, firmware, programmable logic, memory devices (e.g., EEPROMs, ROMs, PROMs, RAMs, SRAMs, etc.), hardware (e.g., integrated circuit chip, Field Programmable Gate Array (FPGA), Application Specific Integrated Circuit (ASIC), etc.), electronic devices, a computer readable non-volatile storage unit (e.g., CD-ROM, floppy disk, hard disk drive, etc.). The article of manufacture may be accessible from a file server providing access to the computer-readable programs via a network transmission line, wireless transmission media, signals propagating through space, radio waves, infrared signals, etc. The article of manufacture may be a flash memory card or a magnetic tape. The article of manufacture includes hardware logic as well as software or programmable code embedded in a computer readable medium that is executed by a processor. In general, the computer-readable programs may be implemented in any programming language, such as LISP, PERL, C, C++, C#, PROLOG, or in any byte code language such as JAVA. The software programs may be stored on or in one or more articles of manufacture as object code.

## Claims

1. A drug delivery apparatus for use with a wearable eye wear device, the apparatus comprising:
a first body (4504) defining a fluid reservoir (4506), the reservoir comprising:
a mist generator (4510);
a supply tube (4508), wherein the supply tube feeds a volume of fluid into the reservoir;
a fluid sensor (4512), the sensor able to detect the presence of fluid in the reservoir;
a second body (4402), the second body comprising:
a releasable fastener, wherein the releasable fastener engages a container (4404);
a first needle (4410) extending into the container, the first needle forming a seal with the container, and able to deliver air into the container;
a second needle (4412) extending into the container and connected to the supply tube, the second needle forming a seal with the container;
a pump (4408), wherein the pump delivers air through the first needle, into the container;
a controller (4302), wherein the controller determines the volume of fluid in the reservoir based on data from the fluid sensor, wherein the controller causes the pump to activate when the volume of fluid is below a predetermined threshold, and wherein activation of the pump causes the contents of the container to flow through the second needle, through the supply tube, and into the reservoir; and
a power source, wherein the power source provides electricity to the pump, the controller, the fluid sensor, and the mist generator.

2. The drug delivery apparatus as described in claim 1, wherein the mist generator (4510) comprises a piezoelectric device.

3. The drug delivery apparatus as described in claim 1 or 2, wherein the releasable fastener comprises a port and wherein the container (4404) releasably engages with the port.

4. The drug delivery apparatus of any one of the preceding claims, wherein the fluid sensor (4512) can detect the level of fluid in the reservoir.

5. The drug delivery apparatus of any one of the preceding claims, wherein the pump (4408) is a manual pump.

6. The drug delivery apparatus of any one of claims 1 to 4, wherein the pump (4408) comprises an electric powered pump.

7. A system for the treating an eye of a patient, the system comprising:
a goggle (4200), the goggle configured to be positioned in close proximity to the eye, the goggle comprising:
an optical sensor (4204), the optical sensor capable of capturing an image of a strain sensor (2614);
a processor (4702), wherein the processor receives data from the optical sensor and determines the amount of strain experienced by the strain sensor; and
a drug delivery apparatus comprising the drug delivery apparatus of claim 1 for dispensing a drug into a volume of space in close proximity to the eye;
wherein the processor determines an intraocular pressure (IOP) value based on the data from the optical sensor; and
wherein the processor triggers the drug delivery apparatus to dispense a drug.

8. The system of claim 7, wherein the goggle further comprises a wireless communication module (4708).

9. The system of claim 7 or 8, wherein the optical sensor (4204) further comprises an image sensor and a light source, optionally wherein the light source is a light emitting diode (LED).

10. The system of any one of claims 7 to 9, wherein the strain sensor is a microfluidic strain sensor further comprising:
a gas reservoir (2630);
a liquid reservoir (2620), optionally wherein the liquid reservoir comprises a plurality of channels capable of deformation when subjected to strain, the deformation causing the channels to increase in volume; and
a channel (2640) having a first end connecting the gas reservoir, and a second end connecting to the liquid reservoir.

11. The system of any one of claims 7 to 10, wherein the strain sensor (2614) is a shaped polymer sheet comprising a plurality of optical markers, the markers having a pattern, the pattern subject to deformation when the strain sensor is subjected to strain.

12. The system of any one of claims 7 to 10, wherein the strain sensor is a pattern of reflected image of the eye.

13. A system for the treating an eye of a patient, the system comprising:
a goggle (4200), the goggle configured to be positioned in close proximity to the eye, the goggle comprising:
a magnetic sensor, the magnetic sensor capable of determining the position of a magnet, optionally wherein the magnet is part of a contact lens platform;
a processor (4602), wherein the processor receives data from the magnetic sensor and determines a change in position of the magnet, the magnet having a first position and a second position; and
a drug delivery apparatus comprising the drug delivery apparatus of claim 1 for dispensing a drug into a volume of space in close proximity to the eye;
wherein the processor determines an IOP value based on the change of position of the magnet between the first and second position; and
wherein the processor triggers the drug delivery apparatus to dispense a drug.

14. The system of any one of claims 7 to 12, wherein the processor (4602) is configured to compare IOP pressure to a predetermined threshold (4606) and activate the drug dispensing system (4608) if the IOP pressure meets the predetermined threshold.

15. The system of any one of claims 7 to 12, wherein the optical sensor (4302) comprises one or more of a camera, a charge-coupled device, a light-sensitive device, a high-definition image sensor, or an electromagnetic sensor.

## Patentansprüche

1. Arzneimittelabgabevorrichtung zur Verwendung mit einer tragbaren Brilleneinheit, wobei die Vorrichtung Folgendes umfasst:
einen ersten Körper (4504), der ein Fluidreservoir (4506) definiert, wobei das Reservoir Folgendes umfasst:
einen Nebelgenerator (4510);
einen Zufuhrschlauch (4508), wobei der Zufuhrschlauch ein Volumen von Fluid in das Reservoir führt;
einen Fluidsensor (4512), wobei der Sensor in der Lage ist, das Vorhandensein von Fluid in dem Reservoir zu erkennen;
einen zweiten Körper (4402), wobei der zweite Körper Folgendes umfasst:
ein lösbares Befestigungsmittel, wobei das lösbare Befestigungsmittel mit einem Behälter (4404) in Eingriff steht;
eine erste Nadel (4410), die sich in den Behälter erstreckt, wobei die erste Nadel eine Dichtung mit dem Behälter bildet und fähig ist, Luft in den Behälter abzugeben;
eine zweite Nadel (4412), die sich in den Behälter erstreckt und mit dem Zufuhrschlauch verbunden ist, wobei die zweite Nadel eine Dichtung mit dem Behälter bildet;
eine Pumpe (4408), wobei die Pumpe Luft durch die erste Nadel in den Behälter abgibt;
eine Steuerung (4302), wobei die Steuerung das Volumen von Fluid in dem Reservoir basierend auf Daten von dem Fluidsensor bestimmt, wobei die Steuerung das Einschalten der Pumpe bewirkt, wenn das Volumen von Fluid unter einem festgelegten Schwellenwert liegt, und wobei das Einschalten der Pumpe das Strömen des Inhalts des Behälters durch die zweite Nadel, durch den Zufuhrschlauch und in das Reservoir bewirkt; und
eine Energiequelle, wobei die Energiequelle Elektrizität für die Pumpe, die Steuerung, den Fluidsensor und den Nebelgenerator bereitstellt.

2. Arzneimittelabgabevorrichtung nach Anspruch 1, wobei der Nebelgenerator (4510) eine piezoelektrische Einheit umfasst.

3. Arzneimittelabgabevorrichtung nach Anspruch 1 oder 2, wobei das lösbare Befestigungsmittel einen Anschluss umfasst und wobei der Behälter (4404) in lösbarem Eingriff mit dem Anschluss steht.

4. Arzneimittelabgabevorrichtung nach einem der vorhergehenden Ansprüche, wobei der Fluidsensor (4512) den Füllstand von Fluid in dem Reservoir erkennen kann.

5. Arzneimittelabgabevorrichtung nach einem der vorhergehenden Ansprüche, wobei die Pumpe (4408) eine manuelle Pumpe ist.

6. Arzneimittelabgabevorrichtung nach einem der Ansprüche 1 bis 4, wobei die Pumpe (4408) eine elektrisch betriebene Pumpe umfasst.

7. System zum Behandeln eines Auges eines Patienten, wobei das System Folgendes umfasst:
eine Korbbrille (4200), wobei die Korbbrille dafür gestaltet ist, in unmittelbarer Nähe zum Auge positioniert zu sein, wobei die Korbbrille Folgendes umfasst:
einen optischen Sensor (4204), wobei der optische Sensor in der Lage ist, ein Bild eines Dehnungssensors (2614) aufzunehmen;
einen Prozessor (4702), wobei der Prozessor Daten von dem optischen Sensor empfängt und den Umfang der Dehnung bestimmt, welcher der Dehnungssensor unterliegt; und
eine Arzneimittelabgabevorrichtung, welche die Arzneimittelabgabevorrichtung nach Anspruch 1 umfasst, zum Ausgeben eines Arzneimittels in ein Volumen eines Raumes in unmittelbarer Nähe zum Auge;
wobei der Prozessor basierend auf den Daten von dem optischen Sensor einen intraokularen Druck (IOP, Augeninnendruck) bestimmt; und
wobei der Prozessor die Arzneimittelabgabevorrichtung auslöst, ein Arzneimittel auszugeben.

8. System nach Anspruch 7, wobei die Korbbrille ferner ein Modul (4708) zur drahtlosen Kommunikation umfasst.

9. System nach Anspruch 7 oder 8, wobei der optische Sensor (4204) ferner einen Bildsensor und eine Lichtquelle umfasst, wobei die Lichtquelle optional eine Leuchtdiode (LED) ist.

10. System nach einem der Ansprüche 7 bis 9, wobei der Dehnungssensor ein mikrofluidischer Dehnungssensor ist, der ferner Folgendes umfasst:
ein Gasreservoir (2630);
ein Flüssigkeitsreservoir (2620), wobei das Flüssigkeitsreservoir optional mehrere Kanäle umfasst, die zur Verformung in der Lage sind, wenn sie einer Dehnung ausgesetzt sind, wobei die Verformung das Vergrößern des Volumens der Kanäle bewirkt; und
einen Kanal (2640) mit einem ersten Ende, das mit dem Gasreservoir verbunden ist, und einem zweiten Ende, das mit dem Flüssigkeitsreservoir verbunden ist.

11. System nach einem der Ansprüche 7 bis 10, wobei der Dehnungssensor (2614) eine geformte Polymerbahn ist, die mehrere optische Markierungen umfasst, wobei die Markierungen ein Muster aufweisen, wobei das Muster einer Verformung ausgesetzt ist, wenn der Dehnungssensor einer Dehnung ausgesetzt ist.

12. System nach einem der Ansprüche 7 bis 10, wobei der Dehnungssensor ein Muster eines reflektierten Bildes des Auges ist.

13. System zum Behandeln eines Auges eines Patienten, wobei das System Folgendes umfasst:
eine Korbbrille (4200), wobei die Korbbrille dafür gestaltet ist, in unmittelbarer Nähe zum Auge positioniert zu sein, wobei die Korbbrille Folgendes umfasst:
einen Magnetsensor, wobei der Magnetsensor in der Lage ist, die Position eines Magneten zu bestimmen, wobei der Magnet optional Teil einer Kontaktlinsenplattform ist;
einen Prozessor (4602), wobei der Prozessor Daten von dem Magnetsensor empfängt und eine Veränderung der Position des Magneten bestimmt, wobei der Magnet eine erste Position und eine zweite Position aufweist; und
eine Arzneimittelabgabevorrichtung, welche die Arzneimittelabgabevorrichtung nach Anspruch 1 umfasst, zum Ausgeben eines Arzneimittels in ein Volumen eines Raumes in unmittelbarer Nähe zum Auge;
wobei der Prozessor basierend auf der Veränderung der Position des Magneten zwischen der ersten und der zweiten Position einen IOP-Wert bestimmt; und
wobei der Prozessor die Arzneimittelabgabevorrichtung auslöst, ein Arzneimittel auszugeben.

14. System nach einem der Ansprüche 7 bis 12, wobei der Prozessor (4602) dazu konfiguriert ist, den IOP-Druck mit einem festgelegten Schwellenwert (4606) zu vergleichen und das Arzneimittelausgabesystem (4608) zu aktivieren, wenn der IOP-Druck den festgelegten Schwellenwert erfüllt.

15. System nach einem der Ansprüche 7 bis 12, wobei der optische Sensor (4302) eines oder mehrere von einer Kamera, einer ladungsgekoppelten Einheit, einer lichtempfindlichen Einheit, einem hochauflösenden Bildsensor und einem elektromagnetischen Sensor umfasst.

## Revendications

1. Appareil d'administration de médicament destiné à être utilisé avec un dispositif de lunetterie portable, l'appareil comprenant :
un premier corps (4504) définissant un réservoir de fluide (4506), le réservoir comprenant :
un générateur de brouillard (4510) ;
un tube d'alimentation (4508), dans lequel le tube d'alimentation introduit un volume de fluide dans le réservoir ;
un capteur de fluide (4512), le capteur étant apte à détecter la présence d'un fluide dans le réservoir ;
un deuxième corps (4402), le deuxième corps comprenant :
un élément de fixation détachable, dans lequel l'élément de fixation détachable vient en prise avec un contenant (4404) ;
une première aiguille (4410) s'étendant dans le contenant, la première aiguille formant un joint d'étanchéité avec le contenant, et étant apte à fournir de l'air dans le contenant ;
une deuxième aiguille (4412) s'étendant dans le contenant et connectée au tube d'alimentation, la deuxième aiguille formant un joint d'étanchéité avec le contenant ;
une pompe (4408), dans lequel la pompe fournit de l'air à travers la première aiguille, dans le contenant ;
un dispositif de commande (4302), dans lequel le dispositif de commande détermine le volume de fluide dans le réservoir sur la base de données provenant du capteur de fluide, dans lequel le dispositif de commande amène la pompe à s'activer lorsque le volume de fluide est au-dessous d'un seuil prédéterminé, et dans lequel l'activation de la pompe amène le contenu du contenant à s'écouler à travers la deuxième aiguille, à travers le tube d'alimentation, et dans le réservoir ; et
une source d'énergie, dans lequel la source d'énergie fournit de l'électricité à la pompe, au dispositif de commande, au capteur de fluide et au générateur de brouillard.

2. Appareil d'administration de médicament tel que décrit dans la revendication 1, dans lequel le générateur de brouillard (4510) comprend un dispositif piézoélectrique.

3. Appareil d'administration de médicament tel que décrit dans la revendication 1 ou 2, dans lequel l'élément de fixation détachable comprend un orifice et dans lequel le contenant (4404) vient en prise de manière détachable avec l'orifice.

4. Appareil d'administration de médicament selon l'une quelconque des revendications précédentes, dans lequel le capteur de fluide (4512) peut détecter le niveau de fluide dans le réservoir.

5. Appareil d'administration de médicament selon l'une quelconque des revendications précédentes, dans lequel la pompe (4408) est une pompe manuelle.

6. Appareil d'administration de médicament selon l'une quelconque des revendications 1 à 4, dans lequel la pompe (4408) comprend une pompe électrique.

7. Système pour le traitement d'un œil d'un patient, le système comprenant :
des lunettes (4200), les lunettes étant conçues pour être positionnées à proximité immédiate de l'œil, les lunettes comprenant :
un capteur optique (4204), le capteur optique pouvant capturer une image d'un capteur de contrainte (2614) ;
un processeur (4702), dans lequel le processeur reçoit des données provenant du capteur optique et détermine la quantité de contrainte subie par le capteur de contrainte ; et
un appareil d'administration de médicament comprenant l'appareil d'administration de médicament selon la revendication 1 pour la distribution d'un médicament dans un volume d'espace à proximité immédiate de l'œil ;
dans lequel le processeur détermine une valeur de pression intraoculaire (PIO) sur la base des données provenant du capteur optique ; et
dans lequel le processeur déclenche l'appareil d'administration de médicament de façon à ce qu'il distribue un médicament.

8. Système selon la revendication 7, dans lequel les lunettes comprennent en outre un module de communication sans fil (4708).

9. Système selon la revendication 7 ou 8, dans lequel le capteur optique (4204) comprend en outre un capteur d'images et une source de lumière, éventuellement dans lequel la source de lumière est une diode électroluminescente (DEL).

10. Système selon l'une quelconque des revendications 7 à 9, dans lequel le capteur de contrainte est un capteur de contrainte microfluidique comprenant en outre :
un réservoir de gaz (2630) ;
un réservoir de liquide (2620), éventuellement dans lequel le réservoir de liquide comprend une pluralité de canaux pouvant se déformer lorsqu'ils sont soumis à une contrainte, la déformation amenant les canaux à augmenter de volume ; et
un canal (2640) ayant une première extrémité reliée au réservoir de gaz, et une deuxième extrémité reliée au réservoir de liquide.

11. Système selon l'une quelconque des revendications 7 à 10, dans lequel le capteur de contrainte (2614) est une feuille polymère façonnée comprenant une pluralité de marqueurs optiques, les marqueurs ayant un motif, le motif étant soumis à une déformation lorsque le capteur de contrainte est soumis à une contrainte.

12. Système selon l'une quelconque des revendications 7 à 10, dans lequel le capteur de contrainte est un motif d'une image réfléchie de l'œil.

13. Système pour le traitement d'un œil d'un patient, le système comprenant :
des lunettes (4200), les lunettes étant conçues pour être positionnées à proximité immédiate de l'œil, les lunettes comprenant :
un capteur magnétique, le capteur magnétique pouvant déterminer la position d'un aimant, éventuellement dans lequel l'aimant est une partie d'une plateforme de lentille de contact ;
un processeur (4602), dans lequel le processeur reçoit des données provenant du capteur magnétique et détermine un changement de position de l'aimant, l'aimant ayant une première position et une deuxième position ; et
un appareil d'administration de médicament comprenant l'appareil d'administration de médicament selon la revendication 1 pour la distribution d'un médicament dans un volume d'espace à proximité immédiate de l'œil ;
dans lequel le processeur détermine une valeur de PIO sur la base du changement de position de l'aimant entre la première et la deuxième position ; et
dans lequel le processeur déclenche l'appareil d'administration de médicament de façon à ce qu'il distribue un médicament.

14. Système selon l'une quelconque des revendications 7 à 12, dans lequel le processeur (4602) est configuré pour comparer la pression PIO à un seuil prédéterminé (4606) et activer le système de distribution de médicament (4608) si la pression PIO atteint le seuil prédéterminé.

15. Système selon l'une quelconque des revendications 7 à 12, dans lequel le capteur optique (4302) comprend un ou plusieurs éléments parmi une caméra, un dispositif à couplage de charge, un dispositif photosensible, un capteur d'images haute définition ou un capteur électromagnétique.
